(19) Europäisches Patentamt

European Patent Office

Office européen des brevets

(11) **EP 1 640 447 A1**

(12) **EUROPEAN PATENT APPLICATION**
published in accordance with Art. 158(3) EPC

(43) Date of publication:
**29.03.2006 Bulletin 2006/13**

(51) Int Cl.:
*C12N 1/20* [(1980.01)]    *A23J 3/10* [(1990.01)]
*A23J 3/34* [(1990.01)]    *C07K 14/47* [(1995.01)]
*C12P 21/06* [(1980.01)]    *A61K 38/55* [(1995.01)]
*A61P 9/12* [(2000.01)]    *C12N 1/20* [(1980.01)]
*C12R 1/46* [(1985.01)]    *C12P 21/06* [(1980.01)]
*C12R 1/46* [(1985.01)]

(21) Application number: **04734041.9**

(22) Date of filing: **20.05.2004**

(86) International application number:
**PCT/ES2004/000227**

(87) International publication number:
**WO 2004/104182 (02.12.2004 Gazette 2004/49)**

(84) Designated Contracting States:
**AT BE BG CH CY CZ DE DK EE ES FI FR GB GR HU IE IT LI LU MC NL PL PT RO SE SI SK TR**
Designated Extension States:
**HR LT LV**

(30) Priority: **21.05.2003 ES 200301186**

(71) Applicant: **Grupo Leche Pascual S.A.**
**28050 Madrid (ES)**

(72) Inventors:
• **MUGUERZA MARQUINEZ, Begona**
**E-09400 Aranda de Duero (ES)**

• **DELGADO DELGADO, Marco Antonio**
**E-09400 Aranda de Duero (ES)**
• **QUIROS DEL BOSQUE, Ana**
**E-09400 Aranda de Duero (ES)**
• **RECIO SANCHEZ, Isidra**
**E-09400 Aranda de Duero (ES)**
• **RAMOS GONZALEZ, Mercedes**
**E-09400 Aranda de Duero (ES)**
• **ALEIXANDRE DE ARTINANO, Ma, Amaya**
**E-09400 Aranda de Duero (ES)**

(74) Representative: **Irache Pereira Tona, Maria**
**C/Joaquin Costa, 51 Esc. A, 2 Izq.**
**28002 Madrid (ES)**

(54) **BIOACTIVE PEPTIDE-PRODUCING STRAINS OF  i ENTEROCOCCUS FAECALIS /i , BIOACTIVE PEPTIDES AND APPLICATIONS THEREOF**

(57) The invention relates to Enterococcus faecalis bacteria which can produce bioactive peptides, such as peptides with angiotensin converting enzyme inhibitor activity and/or antihypertensive activity. The aforementioned bacteria can be used to ferment a substrate comprising one or more proteins, peptides or fragments thereof which contain the amino acid sequence of said bioactive peptides. Moreover, the above-mentioned bacteria and bioactive peptides can be used in the pharmaceutical and food industries for the preparation of pharmaceutical compositions or functional food products.

EP 1 640 447 A1

**SEQUENCE LIST**

<110> Grupo Leche Pascual, S.A.

<120> Bioactive peptide-producing strains of *Enterococcus faecalis*, bioactive peptides and applications thereof

<150> ES P200301186

<151> 2003-05-21

<160> 8

<170> PatentIn version 3.0

<210> 1

<211> 6

<212> PRT

<213> Artificial sequence

<400> 1

Leu His Leu Pro Leu Pro
1               5

<210> 2

<211> 10

<212> PRT

<213> Artificial sequence

<400> 2

Val Leu Gly Pro Val Arg Gly Pro Phe Pro
1               5               10

<210> 3

<211> 7

<212> PRT

<213> Artificial sequence

<400> 3

Leu His Leu Pro Leu Pro Leu

1               5

<210> 4

<211> 9

<212> PRT

<213> Artificial sequence

<400> 4

Val Arg Gly Pro Phe Pro Ile Ile Val

1               5

<210> 5

<211> 6

<212> PRT

<213> Secuencia artificial

<400> 5

Val Val Val Pro Pro Phe

1               5

<210> 6

<211> 11

<212> PRT

<213> Artificial sequence

<400> 6

Leu Thr Gln Thr Pro Val Val Val Pro Pro Phe

1               5               10

<210> 7

<211> 13

<212> PRT

<213> Artificial sequence


<400> 7

Val Leu Gly Pro Val Arg Gly Pro Phe Pro Ile Ile Val
1               5               10


<210> 8

<211> 19

<212> PRT

<213> Artificial sequence


<400> 8

Leu Val Tyr Pro Phe Pro Gly Pro Ile Pro Asn Ser Leu  Pro Gln Asn
1               5               10                  15


Ile Pro Pro


**Description**

**FIELD OF THE INVENTION**

The invention is related to the *Enterococcus faecalis* bacteria which is capable of producing bioactive peptides, particularly peptides showing Angiotensin Converting Enzyme Inhibitor activity (ACEI activity) *in vitro* and/or antihypertensive activity, and to its use in the pharmaceutical and food industries. The invention also refers to said bioactive peptides and their use in the pharmaceutical and food industries.

**BACKGROUND OF THE INVENTION**

High blood pressure is a problem of great public health importance, the transcendence of which is today beyond all doubt due to its prevalence, and also due to the fact that it is one of the main risk factors of cardiovascular diseases. Between 15% and 20% of adults in western countries suffer from it today, and numerous studies have demonstrated the close relationship between untreated high blood pressure and the occurrence of ventricular hypertrophy, congestive heart failure, cerebrovascular accidents, retinopathy, progressive renal failure, dissecting aneurysms, coronary disease and peripheral vascular diseases, such that the prevention and treatment thereof constitutes one of the most used strategies to reduce the risk of cardiovascular diseases (MacMahon S, Peto R, Cutler J *et al.* 1990. Lancet 335: 765-774). All these diseases are also common in the western world. Classical epidemiological studies show that cardiovascular morbidity increases significantly after certain blood pressure values but moreover, cardiovascular and cerebrovascular diseases are the main causes of death in industrialized societies.

Nevertheless, although detection and control of high blood pressure is relatively simple, the truth is that many hypertensive patients are not aware of their disease because they do not show any type of symptoms, and others are diagnosed but receive inadequate treatment. As a result of this, the interest that studies trying to establish new strategies for controlling high blood pressure still have is obvious.

Many hypotheses have been developed to explain the increase in blood pressure levels presented which hypertensive patients have. However, the etiopathogeny of high blood pressure has not yet been completely clarified. The mechanisms by which an increase in blood pressure may occur are multiple, and these mechanisms frequently interact. In any case, the rennin-angiotensin-aldosterone system plays an important role in maintaining blood pressure and in the organic damage secondary to the increase in this variable, and its implication in the modulation of the arterial tone of some hypertensive patients may be important. One of the main components of this system is the Angiotensin Converting Enzyme (ACE) [EC 3.4.15.1] (Ondetti MA, Rubin B, Cushman DW. 1977. Science 196: 441-444), which catalyzes the conversion of angiotensin I, an inactive decapeptide, into angiotensin II, an octapeptide with powerful vasoconstricting activity (Skeggs Lt, Kahn JE, Shumway NP. 1956. J Exp Med 103: 295-299). Also, the ACE catalyzes the inactivation of bradykinin, a molecule that has vasodilating activity.

Therefore, the ACE plays a very important role in regulating blood pressure in the organism, and inhibition thereof may facilitate vasodilation and control of high blood pressure. There is, in fact, a group of ACE inhibitor drugs which are widely used as antihypertensives. Amongst these are included Captopril, Enalapril and Lisinopril. The antihypertensive action of these compounds is enhanced when associated with diuretics. Although relatively uncommon, undesirable effects of the ACE inhibitor drugs may result in a drawback for treating some patients.

On the other hand, milk proteins, in addition to their high nutritional value, may originate bioactive peptides, i.e. peptides that are able to modulate physiological activities important for the health and well being of the consumers. These peptides or protein fragments are inactive when found inside the native protein, but show their biological activity when they are released by chemical or enzymatic hydrolysis.

There are various mechanisms for splitting the milk proteins with the resulting occurrence of peptides. These mechanisms may act in a complementary manner. They may be generated by hydrolysis produced by the processing of the product (temperature rises, pH changes... ), by the use of commercial hydrolytic enzymes or by the use of live proteolytic microorganisms, which are able to release bioactive peptides during the fermenting process of milk, dairy products or any protein substrate.

Research carried out up to now confirm various biological activities found in peptides derived from milk proteins and their potential applications (Kayser H, Meisel H. 1996. FEBS Letters 383: 18-20; Tome D, Debabbi H. 1998. Int Dairy J 8: 383-392; Gaucheron F, Mollé D, Briard V, Léonil J. 1999. Int Dairy J 9: 515-521; Cross ML, Gill HS. 2000. Br J Nutr 84: 81S-89S; Shah NP. 2000. Br J Nutr 84: 3S-10S). Amongst the biological activities described for these peptides the following stand out: antihypertensive activity, opiate activity, immunomodulatory effect, antimicrobial activity, antithrombotic activity and the capacity to bind minerals. Peptides with antihypertensive activity have been obtained from enzymatic hydrolysates of different food proteins of animal and plant origin, but milk proteins, both caseins as whey proteins, constitute the main source of peptides with antihypertensive activity (Yamamoto N. 1997. Biopolymers 43: 129-134).

Within the research on functional peptides with ACE inhibitor activity (ACEI activity) and/or antihypertensive activity, the work of the Yamamoto team et al. stands out (Nakamura Y, Yamamoto N, Sakai K *et al.* 1995. J Dairy Sci 78: 777-783; Nakamura Y, Yamamoto N, Sakai K *et al.* 1995. J Dairy Sci 78: 1253-1257). These researchers studied the effect exerted by milk fermented by different strains of *Lactobacillus helveticus* and *Saccharomyces cerevisiae* on blood pressure. They identified various peptides originating from the fermented milk that showed ACEI activity *in vitro,* and they found that after the fermentation process, the fermented milk reduced blood pressure in spontaneously hypertensive rats (SHR). However, the fermented milk did not modify the blood pressure in Wistar-Kyoto rats (WKY), which are the normotensive control of SHR rats. Later, said researchers demonstrated that the fermented milk showed antihypertensive effects in humans (Hata Y, Yamamoto N, Ohni M, *Y et al.* 1996. Am J Clin Nutr 64: 767-771).

The effect of milk fermented by *Lactobacillus helveticus* which reduces blood pressure in SHR rats (Sipola M, Finckenberg P, Korpela R; Vapaatalo H, Nurminen Ml. 2002. J Dairy Res 69: 103-111) and in hypertensive humans (Seppo LM, Kerojoki O, Suomalainen H, Korpela R. 2002. Milchwissenschaft 57: 124-127; Seppo LM, Jauhiainen T, Poussa T, Korpela R. 2003. Am J Clin Nutr 77: 326-330) has recently been disclosed. The results of this research have been translated into the commercialization of some fermented milks and yogurt-type fermented dairy products containing antihypertensive peptides (Calpis®, Calpis Food Industry Co., Ltd, Tokyo, Japan; Evolus®, Valio, Finland).

Additionally, peptides with ACEI activity have been isolated and characterized in milk fermented with *Lactobacillus delbrueckii* and *Lactococcus lactis* (Gobbetti M, Ferranti P, Smacchi E *et al.* 2000. Appl Environ Microbiol 66: 3898-3904) and also in Manchego cheese (aged sheep's milk cheese) (Gómez-Ruiz JA, Ramos M, Recio I. 2002. Int Dairy J 12: 697-706).

The LHLPLP peptide has been described as a peptide showing ACEI activity when said activity is assessed *in vitro.* However, its hypertensive activity has not been demonstrated (Kohmura M, Nio N, Kubo K *et al.* 1989. Agric Biol. Chem 53:2107-2114). In relation to this it is worth mentioning that many peptides that are characterized as strong inhibitors of the ACE *in vitro* show, however, a very small or null effect on this enzyme when tested *in vivo* (Maruyama S, Mitachi H, Awaya J *et al.* 1987. Agric Biol Chem 51: 2557-2561; Maeno M, Yamamoto N, Takano T. 1996. J Dairy Sci 79: 1316-1321). The opposite case may also occur, that is, peptides showing little ACEI activity *in vitro* may be able to acquire this activity *in vivo* due to the action of the digestive enzymes upon them (Maeno M, Yamamoto N, Takano T. 1996. J Dairy Sci 79: 1316-1321). All these results show the need for proving the antihypertensive efficacy of the peptides showing ACEI activity *in vitro,* the effect of these peptides on the blood pressure of hypertensive animals must first be studied, and if appropriate, the attempt will then be made to confirm their antihypertensive effect in humans (Darragh AJ. 2002. Bull IDF 375:25-31).

On the other hand, the antihypertensive potential of orally administered peptides depends on their ability to reach their action sites without being degraded, and consequently inactivated, by digestive tract enzymes. Therefore, resistance to said enzymes is a prerequisite for a hypertensive effect *in vivo* after oral ingestion of said peptides.

## SUMMARY OF THE INVENTION

Although bioactive peptides from different substrates are known, and also processes to produce such bioactive peptides based on fermentation of a substrate by certain microorganisms, given the increasing interest and demand of functional food products incorporating bioactive peptides, the search for new bioactive peptides as well as the development of alternative processes to produce said peptides is timely, convenient and almost necessary. Advantageously, said bioactive peptides must be resistant to the action of digestive tract enzymes or rather be precursors of active forms (that may subsequently be released by the action of gastrointestinal enzymes) so that they may be orally administered.

This invention provides bioactive peptides, particularly peptides showing ACEI activity *in vitro* and/or with antihypertensive activity when administered by intragastric probe. Therefore, they may be orally administered and used in the elaboration of functional food products and/or in the elaboration of pharmaceutical compositions for the treatment and/or prophylaxis of high blood pressure. It has also been found that the *Enterococcus faecalis* bacteria is capable of producing said bioactive peptides by proteolysis of proteins the amino acid sequences of which contain the amino acid sequences of said bioactive peptides.

Therefore, an aspect of this invention is related to the *Enterococcus faecalis* bacteria having the capability of producing bioactive peptides, particularly peptides showing ACEI activity *in vitro* and/or with antihypertensive activity. In a particular embodiment, said bacteria is a strain of *E. faecalis* deposited in the CECT (Colección Española de Cultivos Tipo - *Spanish Type Culture Collection)* chosen from the strain identified as *E. faecalis* CECT 5727, the strain identified as *E. faecalis* CECT 5728, the strain identified as *E. faecalis* CECT 5826 and the strain identified as *E. faecalis* CECT 5827. Said bacteria can be used in the production of bioactive peptides useful in the food and pharmaceutical industries.

In another aspect, the invention is related to a bacterial preparation containing *E. faecalis* CECT 5727, *E. faecalis* CECT 5728, *E. faecalis* CECT 5826 and/or *E. faecalis* CECT 5827 and optionally one or more different microorganisms. In a particular embodiment, said bacterial preparation can be found in the form of lyophilized powder or in a capsule.

In another aspect, the invention is related to a process for producing a bioactive peptide by fermentation of a substrate containing a protein, peptide or fragment thereof containing said bioactive peptide by *E. faecalis.* In a particular embodiment said bioactive peptide is a peptide showing ACEI activity *in vitro* and/or antihypertensive activity, chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof and mixtures thereof, and said bacteria is chosen from *E. faecalis* CECT 5727, *E. faecalis* CECT 5728, *E. faecalis* CECT 5826, *E. faecalis* CECT 5827 and mixtures thereof.

In another aspect, the invention is related to the use of *E. faecalis* CECT 5727, *E. faecalis* CECT 5728, *E. faecalis* CECT 5826 and/or *E. faecalis* CECT 5827 in the food industry. In a particular embodiment, the invention is related to the use of said strains in the preparation of a food product, for example, a dairy product, optionally fermented and/or subjected to heat treatment, or a drink. The food products comprising said bacteria or bacterial preparation, or which have been prepared using said bacteria or bacterial preparation, also constitute a further aspect of this invention.

In another aspect, the invention is related to the use of *E. faecalis* CECT 5727, *E. faecalis* CECT 5728, *E. faecalis* CECT 5826 and/or *E. faecalis* CECT 5827 as a therapeutic substance, particularly to the use of said bacteria in treating and/or preventing high blood pressure in all its forms in a human being or in an animal, as well as to its use in treating and/or preventing any disorder associated with high blood pressure in a human being or an animal.

In another aspect, the invention is related to the use of *E. faecalis* CECT 5727, *E. faecalis* CECT 5728, *E faecalis* CECT 5826 and/or *E. faecalis* CECT 5827 in obtaining bioactive peptides, particularly in obtaining peptides showing ACEI activity *in vitro* and/or antihypertensive activity. In a particular embodiment, said bioactive peptide is chosen from a group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof.

In another aspect, the invention is related to the use of *E. faecalis* CECT 5727, *E. faecalis* CECT 5728, *E. faecalis* CECT 5826 and/or *E. faecalis* CECT 5827 in the preparation of an antihypertensive product. In a particular embodiment, said antihypertensive product comprises the antihypertensive peptide identified by SEQ. ID. NO: 1, derivatives thereof and/or pharmaceutically acceptable salts thereof.

In another aspect, the invention is related to a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof and pharmaceutically acceptable salts thereof.

In another aspect, the invention is related to a composition comprising a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof.

In another aspect, the invention is related to a pharmaceutical composition comprising a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof. In a particular embodiment, said pharmaceutical composition is an antihypertensive pharmaceutical composition.

In another aspect, the invention is related to a the use of a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof, in the elaboration of a drug for treating and/or preventing high blood pressure in all its forms in a human being or in an animal, as well as to the use thereof in treating and/or preventing any disorder associated with high blood pressure in a human being or in an animal.

In another aspect, the invention is related to obtaining functional foods with antihypertensive activity, fermented milks, for example, containing an antihypertensive peptide, such as the peptide identified by SEQ. ID. NO: 1, derivatives thereof and/or pharmaceutically acceptable salts thereof.

In another aspect, the invention is related to a functional food product comprising a food product and a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof.

In another aspect, the invention is related to a food additive or to a food supplement comprising a peptide chosen from the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof.

Finally, in another aspect, the invention is related to a method for treating and/or preventing high blood pressure in all its forms, or disorders associated with high blood pressure in a subject such as a human or an animal, comprising the administration to said subject of an effective amount of an antihypertensive pharmaceutical composition provided by this invention.

Other features of the invention will be subsequently developed in the description of the invention which will be detailed below.

**BRIEF DESCRIPTION OF THE FIGURES**

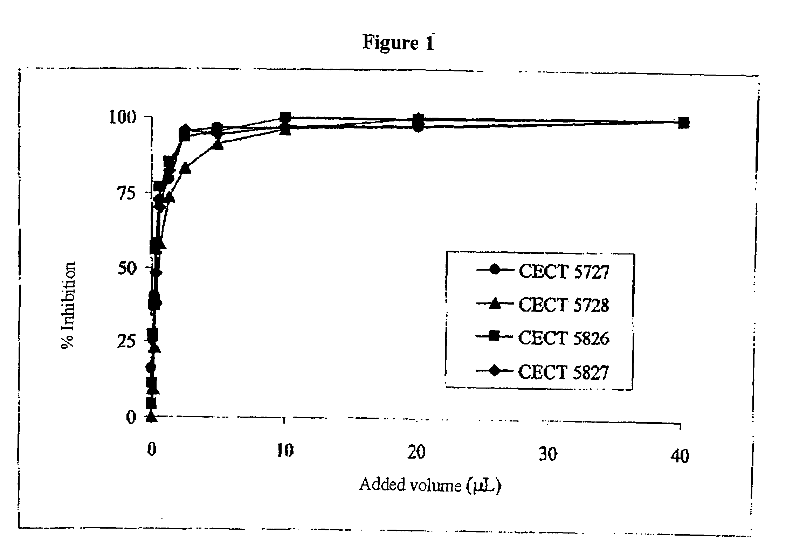

Figure 1 is a graph representing the percentage of ACE inhibition obtained according to the added volume of supernatant or whey fermented with *E. faecalis* CECT 5727 (•), with *E. faecalis* CECT 5728 (▲), with *E. faecalis* CECT 5826 (■) or with *E. faecalis* CECT 5827 (♦).

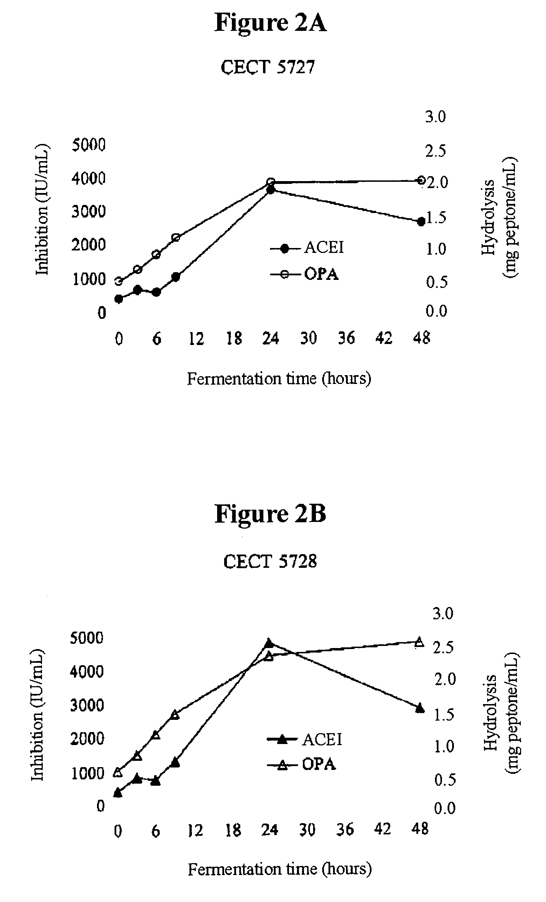

Figure 2A is a graph showing hydrolysis evolution measured by the OPA method and expressed as mg peptone/mL (o), and the whey dilution activity producing a 50% inhibition of enzyme activity expressed in inhibition units per volume (IU/mL), (•), according to the fermentation time for the milk fermented by *E. faecalis* CECT 5727.

Figure 2B is a graph showing hydrolysis evolution measured by the OPA method and expressed as peptone/mL (Δ), and the whey dilution activity producing a 50% inhibition of enzyme activity expressed in inhibition units per volume (IU/mL), (▲), according to fermentation time for the milk fermented by *E. faecalis* CECT 5728.

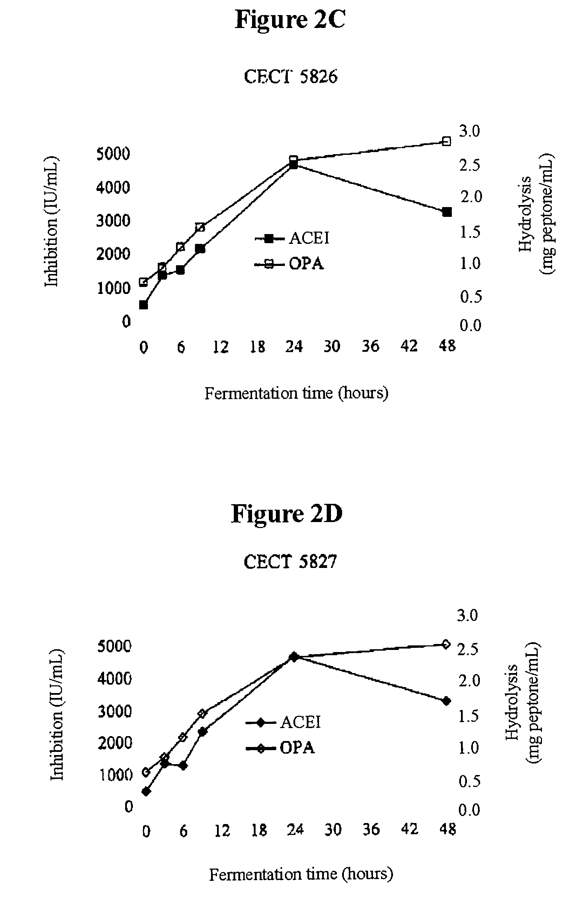

Figure 2C is a graph showing hydrolysis evolution measured by the OPA method and expressed as peptone/mL (□), and the whey dilution activity producing a 50% inhibition of enzyme activity expressed in inhibition units per volume (IU/mL), (■), according to fermentation time for the milk fermented by *E. faecalis* CECT 5826.

Figure 2D is a graph showing hydrolysis evolution measured by the OPA method and expressed as peptone/mL (◊), and the whey dilution activity producing a 50% inhibition of enzyme activity expressed in inhibition units per volume (IU/mL), (♦), according to the fermentation time for the milk fermented by *E. faecalis* CECT 5827.

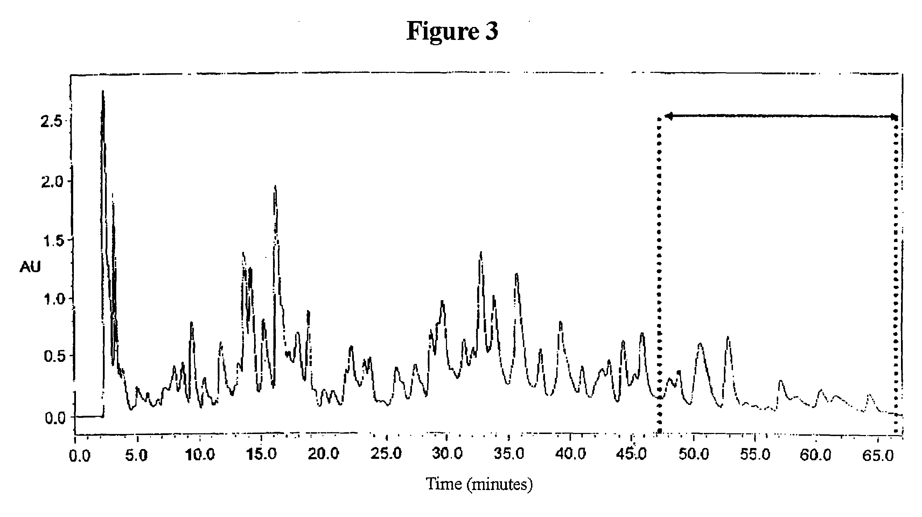

Figure 3 is a reversed-phase HPLC chromatogram at semipreparative scale of the active supernatant obtained after centrifugation and ultrafiltration through a 3,000 Da pore size membrane of the milk fermented by *E. faecalis* CECT 5727. The peptides with ACEI activity elute with retention times exceeding 47 minutes.

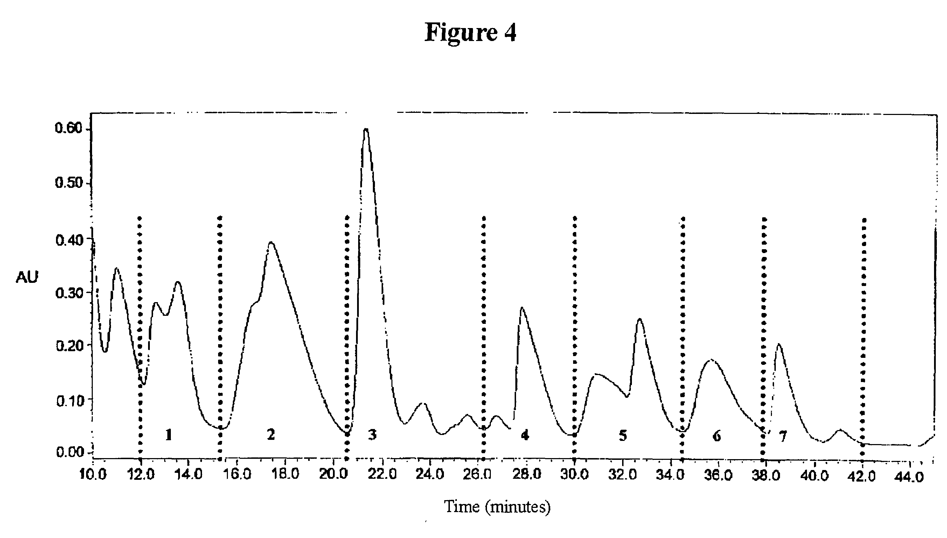

Figure 4 is a reversed-phase HPLC chromatogram at semipreparative scale of the active fraction obtained from the first separation by HPLC. The peptides with ACEI activity were collected in 7 fractions eluting between minute 12 and minute 42.

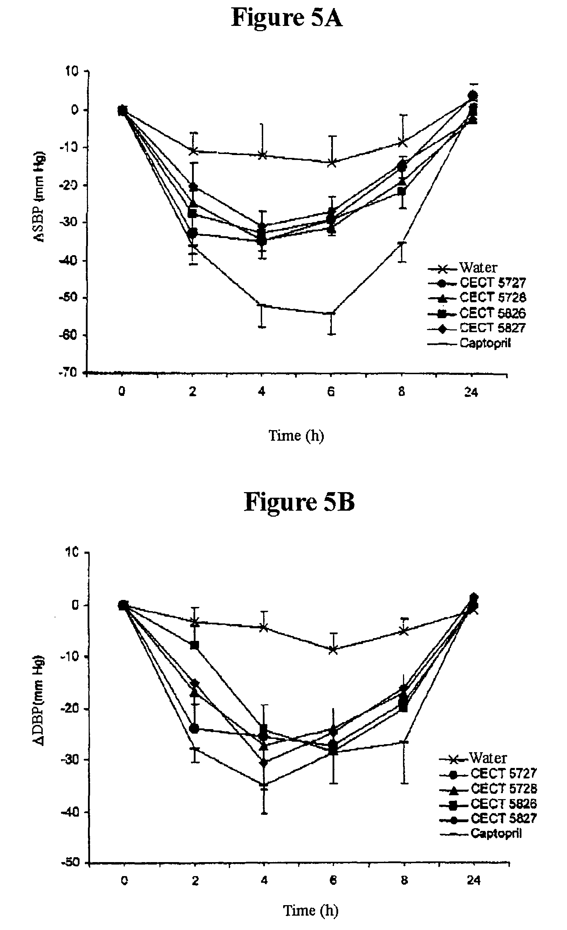

Figure 5A is a graph showing the decrease in systolic blood pressure (SBP) obtained in spontaneously hypertensive rats that were administered 1 mL of water (X), 5 mL/kg of whey fermented with *E. faecalis* CECT 5727 (•), 5 mL/kg of whey fermented with *E. faecalis* CECT 5728 (▲), 5 mL/kg of whey fermented with *E. faecalis* CECT 5826 (■), 5 mL/kg of whey fermented with *E. faecalis* CECT 5827 (♦) or 50 mg/kg of Captopril (-), according to the time elapsed since administration. The data represents the mean ± SEM for a minimum of 7 animals. Unless otherwise indicated, all indicated doses in this description refer to the body weight of the animal.

Figure 5B is a graph showing the decrease in diastolic blood pressure (DBP) obtained in spontaneously hypertensive rats that were administered 1 mL of water (X), 5 mL/kg of when fermented with *E. faecalis* CECT 5727 (•), 5 mL/kg of whey fermented with *E. faecalis* CECT 5728 (▲), 5 mL/kg of whey fermented with *E. faecalis* CECT 5826 (■), 5 mL/kg of whey fermented with *E. faecalis* CECT 5827 (♦) or 50 mg/kg of Captopril (-), according to the time elapsed since administration. The data reshows the mean ± SEM for a minimum of 7 animals.

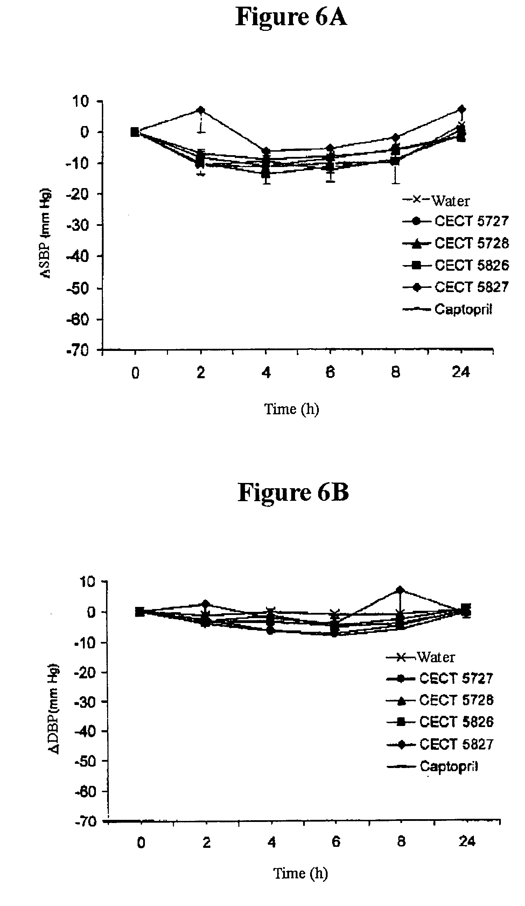

Figure 6A is a graph showing the variation of systolic blood pressure (SBP) obtained in Wistar-Kyoto rats that were administered 1 mL of water (X), 5 mL/kg of whey fermented with *E. faecalis* CECT 5727 (•), 5 mL/kg of whey fermented with *E. faecalis* CECT 5728 (▲), 5 mL/kg of whey fermented with *E. faecalis* CECT 5826 (■), 5 mL/kg of whey fermented with *E. faecalis* CECT 5827 (♦) or 50 mg/kg of Captopril (-), according to the time elapsed since administration. The data represents the mean ± SEM for a minimum of 7 animals.

Figure 6B is a graph showing the variation of diastolic blood pressure (DBP) obtained in Wistar-Kyoto rats that were administered 1 mL of water (X), 5 mL/kg of whey fermented with *E. faecalis* CECT 5727 (•), 5 mL/kg of whey fermented with *E. faecalis* CECT 5728 (▲), 5 mL/kg of whey fermented with *E. faecalis* CECT 5826 (■), 5 mL/kg of whey fermented with *E. faecalis* CECT 5827 (♦) or 50 mg/kg of Captopril (-), according to the time elapsed since administration. The data represents the mean ± SEM for a minimum of 7 animals.

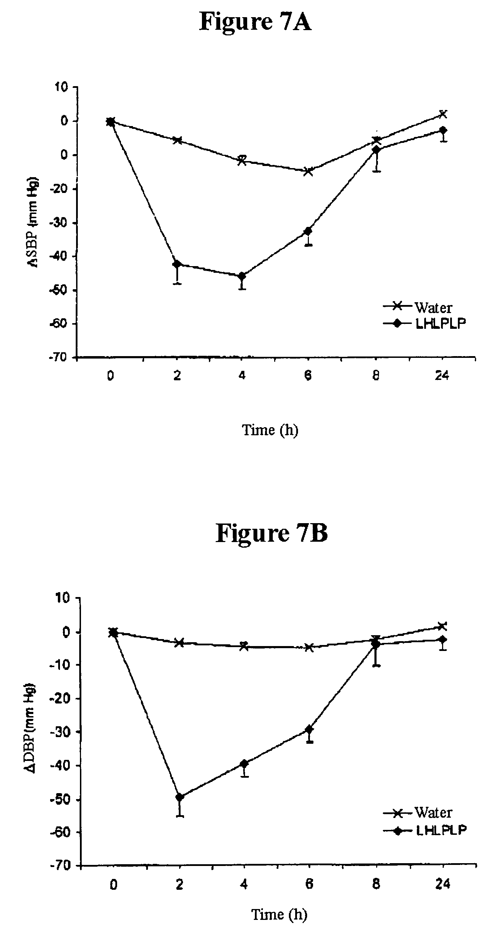

Figure 7A is a graph showing the decrease in systolic blood pressure (SBP) obtained in spontaneously hypertensive rats that were administered 1 mL of water (X) or 2 mg/kg of the LHLPLP peptide (♦), according to the time elapsed since administration. The data represents the mean ± SEM for a minimum of 9 animals.

Figure 7B is a graph showing the decrease in diastolic blood pressure (DBP) obtained in spontaneously hypertensive rats that were administered 1 mL of water (X) or 2 mg/kg of the LHLPLP peptide (♦), according to the time elapsed since administration. The data represents the mean ± SEM for a minimum of 9 animals.

## DETAILED DESCRIPTION OF THE INVENTION

In a first aspect, the invention is related to strains of *Enterococcus faecalis* with proteolytic activity and ability to produce bioactive peptides, particularly peptides showing ACEI activity *in vitro* and/or with antihypertensive activity. In a particular embodiment, the invention provides a strain of *E. faecalis* deposited at the CECT chosen from the strain identified as *E. faecalis* CECT 5727, the strain identified as *E. faecalis* CECT 5728, the strain identified as *E. faecalis* CECT 5826 and the strain identified as *E. faecalis* CECT 5827. The first two strains of *E. faecalis* were deposited at the CECT on October 10th, 2002, their access number being as indicated, and the second two strains were deposited at the CECT on July 17th, 2003, their access number being as indicated.

The strains of *E. faecalis* CECT 5727, *E. faecalis* CECT 5728, *E. faecalis* CECT 5826 and *E. faecalis* CECT 5827 have been chosen from microorganisms isolated from raw bovine milk of different origins, after having been subjected to a selection process based on the presence of ACEI activity exceeding 70% in the supernatants after centrifugation (whey) of the milks fermented with said microorganisms. The strains were isolated and subsequently characterized by their fermenting and biochemical profile. In Example 1 the isolation and characterization of said strains *E. faecalis* CECT 5727, *E. faecalis* CECT 5728, *E. faecalis* CECT 5826 and *E. faecalis* CECT 5827 are described in detail.

The strain of *E. faecalis* CECT 5727 shows, among others, the following characteristics:

- Colony characteristics:

    1) Growth in M 17, optionally in MRS
    2) Shape: round
    3) Size: 1 mm (in 24 hours)
    4) Color: white
    5) Appearance: shiny
    6) Elevation: flat
    7) Edge: irregular

- Morphological characteristics:

    1) Shape: coccal
    2) Motility: none
    3) Spore formation: no
    4) GRAM stain: positive

- Physiological properties:

    1) Catalase production: negative
    2) Aerobic growth, anaerobiosis optional
    3) Biochemical profile:

        Arginine dihydrolase: +
        β-Glucosidase: +
        β-Glucuronidase: -
        α-Galactosidase: -
        β-Galactosidase -
        Alkaline Phosphatase: -
        Ribose (Acidification): +
        Mannitol (Acidification): +
        D-sorbitol (Acidification):+
        Lactose (Acidification): +
        Trehalose (Acidification): +
        Raffinose (Acidification): -
        Sucrose (Acidification): +
        L-arabinose (Acidification): -
        D- arabitol (Acidification): -
        Cyclodextrin (Acidification): +
        Acetoin Production de: +
        Alanyl-Phenylalanyl-Proline Arylamidase: -

Pyroglutamic Acid Arylamidase: +
N-Acetyl-beta Glucosaminidase.: +
Glycyl- Tryptophan Arylamidase: +
Hippurate Hydrolysis : -
Glycogen (Acidification): -
Pullulan (Acidification): -
D-maltose (Acidification): +
Melibiose (Acidification): -
Melezitose (Acidification): +
Methyl β-D-glucopyranoside (Acidification): +
Tagatose (Acidification): +
β-mannosidase: +
Urease: -

The strain *E. faecalis* CECT 5728 shows morphological characteristics, physiological properties and fermentative and biochemical profile similar to those of strain *E. faecalis* CECT 5727 with the following exceptions:

Appearance of the colony: milky
Colony elevation: raised
Colony edge: smooth.
β-Glycosidase: -
N-Acetyl-beta Glucosaminidase: -
Methyl β-D-glucopyranoside (Acidification): -
β-mannosidase: -

The strain *E. faecalis* CECT 5826 shows morphological characteristics, physiological properties and fermentative and biochemical profile similar to those of strain *E. faecalis* CECT 5727 with the following exceptions:

Colony size: 2-3 mm (in 24 hours)
Colony margin: smooth.
N-Acetyl-beta Glucosaminidase.: -
Glycyl- Tryptophan Arylamidase: -
Methyl β-D-glucopyranoside (Acidification): -
β-mannosidase: -

The strain *E. faecalis* CECT 5827 shows morphological characteristics, physiological properties and fermentative and biochemical profile similar to those of strain *E. faecalis* CECT 5727 with the following exceptions:

Colony size: 4 mm (en 24 hours)
Colony appearance: milky.
Colony elevation: raised.
N-Acetyl-beta Glucosaminidase: -
Glycyl- Tryptophan Arylamidase: -
Methyl β-D-glucopyranoside (Acidification): -
β-mannosidase: -

Strains *E. faecalis* CECT 5727, *E. faecalis* CECT 5728, *E. faecalis* CECT 5826 and *E. faecalis* CECT 5827 are proteolytic strains capable of producing bioactive peptides, particularly peptides with ACEI activity and/or with antihypertensive activity, from an appropriate substrate comprising one or more proteins, peptides or fragments of said proteins and peptides, which contain the amino acid sequence of said bioactive peptides of interest, by means of proteolytic digestion. Said substrate is generally a source of proteins, peptides or fragments thereof, of animal or plant origin or from microorganisms, comprising one or more proteins, peptides or fragments thereof containing the amino acid sequence of said bioactive peptides of interest. The proteolytic activity of the strains *E. faecalis* CECT 5727, *E. faecalis* CECT 5728, *E. faecalis* CECT 5826 and *E. faecalis* CECT 5827 has been tested by the OPA (o-phthalaldehyde) method using casein peptone for the calibration line (Church FC, Swaisgood HE, Porter DH, Catgnani L. 1983. J Dairy Sci 66: 1219-1227) [Example 2]. The ACEI activity of the substrates fermented with said strains can be determined by the method described in Example 1.2.

In a particular embodiment said bioactive peptides produced by strains *E. faecalis* CECT 5727, *E. faecalis*

CECT 5728, *E. faecalis* CECT 5826 and *E. faecalis* CECT 5827 are the peptides identified with the amino acid sequences shown in SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7 and SEQ. ID. NO: 8, some of which show ACEI activity and/or antihypertensive activity (Examples 3 and 4).

Therefore, in another aspect, the invention is related to the use of a strain of *E. faecalis,* particularly a strain of *E. faecalis* chosen from *E. faecalis* CECT 5727, *E. faecalis* CECT 5728, *E. faecalis* CECT 5826 and *E. faecalis* CECT 5827 and mixtures thereof in obtaining bioactive peptides, particularly peptides with ACEI activity and/or antihypertensive activity, chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof.

As used in this description the term "derivative" includes any chemical derivative of said peptides containing a chemical modification that does not adversely affect their biological activity, for example, their ACEI and/or antihypertensive activity; advantageously, said chemical modification will be a modification such that their biological activity increases. In a particular embodiment, said derivative is a peptide derivative containing a phosphorylation or sulfhydryl group at the amine terminal end or any other chemical modification that does not reduce the biological activity of the peptide. As is well known, the presence of the sulfhydryl or phosphoric group generally increases the activity of ACE inhibitor peptides (Ondetti MA, Rubin B, Cushman DW. 1977. Science 196: 441-444). The derivatives of the peptides provided by this invention can be obtained by conventional methods that are well known by persons skilled in the art by reacting the peptide provided by the invention with a reagent which provides the desired chemical modification.

The term "pharmaceutically acceptable salts" includes salts commonly used to form metal salts or acid addition salts. The nature of the salt is not critical as long as it is pharmaceutically acceptable. Pharmaceutically acceptable salts of the peptides provided by this invention can be obtained from organic or inorganic acids or bases. Said salts can be obtained by conventional methods that are well known by persons skilled in the art by reacting the appropriate acid or base with the peptide provided by the invention.

More specifically, the invention provides a process for producing a bioactive peptide which comprises fermenting a substrate comprising one or more proteins, peptides or fragments thereof containing the amino acid sequence of said bioactive peptide with a strain of *E. faecalis* and, if so desired, isolating the bioactive peptide and/or converting it into a derivative and/or a pharmaceutically acceptable salt.

In a particular embodiment, said bioactive peptide is a peptide with ACEI activity and/or antihypertensive activity, such as a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof.

In another particular embodiment, said strain of *E. faecalis* is chosen from the group formed by *E. faecalis* CECT 5727, *E. faecalis* CECT 5728, *E. faecalis* CECT 5826 and *E. faecalis* CECT 5827 and mixtures thereof. Said strain may be used as a pure or substantially pure culture or as a mixed culture, in the form of a bacterial preparation containing *E. faecalis* CECT 5727, *E. faecalis* CECT 5728, *E. faecalis* CECT 5826 and/or *E. faecalis* CECT 5827, optionally along with one or more different microorganisms such as *Streptococcus thermophilus, Lactobacillus bulgaricus, Lactobacillus casei, Lactobacillus acidophilus, Lactobacillus helveticus* or any other dairy bacteria. Said bacterial preparation, hereinafter bacterial preparation of the invention, containing *E. faecalis* CECT 5727, *E. faecalis* CECT 5728, *E. faecalis* CECT 5826 and/or *E. faecalis* CECT 5827 and, optionally one or more of said different microorganisms, constitutes a further aspect of this invention. The bacterial preparation of the invention may be presented in any suitable form; however, in a particular embodiment, said bacterial preparation is found in dehydrated, lyophilized or capsule form

The substrate to be fermented is generally a source of proteins, peptides or fragments thereof, comprising one or more proteins, peptides or fragments thereof containing the amino acid sequence of said bioactive peptide. Said substrate can be of animal origin, for example milk, eggs, muscles from birds, fish or mammals, etc., or of plant origin, for example protein extracts of soy, corn, wheat, nuts and dried fruits, etc., or it may even come from a microorganism, for example bacteria, yeasts, fungi, etc. In a particular embodiment said substrate comprises milk or a dairy product, optionally fermented, containing casein, β-casein, for example, such as raw milk, whole milk, skimmed or semi-skimmed milk, milk powder (after reconstitution thereof), yogurt, butter, cheese, etc., or alternatively a soy protein extract. When the substrate is milk, this may come from any mammal, for example a cow, sheep, goat, buffalo, etc. The sequence of the peptides SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7 and SEQ. ID. NO: 8 can be found in the sequence of bovine β-casein (see Table 3, Example 3).

The conditions for fermenting said substrate by *E faecalis* are chosen so that they are suitable for said bacteria to perform its proteolytic activity on the substrate, such that the bioactive peptides are produced. The fermenting process can be ended by any suitable method, for example by modifying the pH of the medium, applying a heat treatment, etc. The choice of the suitable conditions, such as temperature, pH and/or aeration, as well as of the conditions for ending the fermentation, are part of the general knowledge of a person skilled in the art. In general, when the substrate is liquid, milk, for example, a bacterial inoculum with a suitable bacterial density is added to the substrate and is left to ferment during a suitable time period at the suitable temperature and pH. When the substrate is solid or substantially solid, the substrate is mixed with a suitable liquid, such as sterilized water, it is mixed until obtaining a homogenous mixture, the bacterial inoculum is added and it is left to ferment in suitable conditions. The pH of the fermenting medium is adjusted in order to promote bacterial proteolytic activity, either before the start of fermentation or during fermentation, by adding an acid or a base, according to what is necessary. Fermentation can be repeated a variable number of times adding either fresh bacterial inoculums or bacterial inoculums from previous fermentations on the substrate.

After fermenting the substrate a mixture of bioactive peptides is obtained. In a particular embodiment said bioactive peptide is a peptide with ACEI activity and/or with antihypertensive activity, such as a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, and mixtures thereof. The measurement of ACEI activity of the fermented products can be done by conventional methods as described in Example 1.2. If so desired, said bioactive peptides can be isolated and purified by conventional methods and/or converted into derivatives and/or pharmaceutically acceptable salts by conventional methods.

In a particular embodiment, the fermentation of the substrate with *E. faecalis* is performed by means of a process comprising a first fermentation at a temperature comprised between 10°C and 50°C, typically around 30°C, for a period of time comprised between 5 and 72 hours, typically around 24 hours, with an inoculum load with a bacterial density comprised between 10 and $10^{12}$, preferably between $10^7$ and $10^9$, bacteria per 100 mL of substrate, followed by a second fermentation at a temperature comprised between 10°C and 50°C, typically around 30°C, for a period of time comprised between 5 and 72 hours, typically around 48 hours, with an inoculum load from the first fermentation comprised between 0.01% and 90% by weight, typically around 3% by weight. Example 3 describes the isolation and characterization of bioactive peptides with ACEI activity and/or with antihypertensive activity chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, and mixtures thereof, by fermentation of bovine milk with *E. faecalis* CECT 5727, *E. faecalis* CECT 5728, *E. faecalis* CECT 5826 or with *E. faecalis* CECT 5827.

The fermented substrate containing the bioactive peptides, particularly the fermented substrate containing a peptide with ACEI activity and/or with antihypertensive activity chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof, can be used as an antihypertensive agent. Said fermented product, if so desired, can be pasteurized or dried and used in the form of a powder or a lyophilized preparation, and it can be used in the elaboration of various products, amongst which are food products, including functional food products. The optionally pasteurized fermented substrate constitutes a further aspect of this invention.

Therefore, in another aspect the invention is related to the use of a strain of *E. faecalis* chosen from *E. faecalis* CECT 5727, *E. faecalis* CECT 5728, *E. faecalis* CECT 5826, *E. faecalis* CECT 5827 and mixtures thereof as a therapeutic substance, particularly as a therapeutic substance for treating and/or preventing high blood pressure in all its forms in a human being or in an animal, and for treating and/or preventing any disorder associated with high blood pressure in a human being or in an animal, such as ventricular hypertrophy, congestive heart failure, cerebrovascular accidents, retinopathy, progressive renal failure, dissecting aneurysms, coronary disease and peripheral vascular diseases.

In the same manner, in another aspect the invention is related to the use of a strain of *E. faecalis,* particularly with a strain of *E. faecalis* chosen from *E. faecalis* CECT 5727, *E. faecalis* CECT 5728, *E. faecalis* CECT 5826, *E. faecalis* CECT 5827 and mixtures thereof, or of a bacterial preparation of the invention in the food industry, for example in the preparation of a food product. Therefore, in another aspect, the invention provides a food product comprising a strain of *E. faecalis,* particularly a strain of *E. faecalis* chosen from *E. faecalis* CECT 5727, *E. faecalis* CECT 5728, *E. faecalis* CECT 5826, *E. faecalis* CECT 5827 and mixtures thereof, or rather a bacterial preparation, or a food product that has been prepared using said strain of *E. faecalis* of the invention or said bacterial preparation of the invention.

The term "food product" is used in this description in its broadest sense, including any type of product, in any presentation form, for example, in solid, liquid or gel form, that can be ingested by a mammal, for example, a food or nutritional substance. The term "food" as used in this description includes substances and compositions that can be used or prepared for their use as food for human beings and animals, including substances that can be used for their preparation, for example, oils, food ingredients and food additives. As an illustration, the term "food" includes drinks (for example soft drinks, carbonated drinks, etc.), infusion-type foods (for example vegetables, etc.), sauces, condiments, salad dressings, fruit juices, syrups, desserts (for example puddings, gelatins, fillings, baked products, frozen desserts such as ice-creams, sorbets, etc.), ice creams, lightly frozen products, sweets (for example candies, etc.), chewing gum, etc.

In a particular embodiment, said food product is a dairy product, optionally fermented and/or subjected to heat treatment, pasteurized, for example, such that it is free of viable microorganisms, such as milk, yogurts, etc. or a drink, for example, a drink with whey, a fruit drink, a beer, etc.

In another aspect, the invention refers to a bioactive peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ.

ID. NO: 7, SEQ. ID. NO: 8, derivatives thereof and pharmaceutically acceptable salts thereof. The terms "derivatives" and "pharmaceutically acceptable salts" have been defined above.

The peptides identified by SEQ. ID. NO: 2, SEQ. ID. NO: 3, SEQ. ID. NO: 4, SEQ. ID. NO: 5, SEQ. ID. NO: 6, SEQ. ID. NO: 7 and SEQ. ID. NO: 8, derivatives thereof and pharmaceutically acceptable salts thereof, as well as the derivatives and the pharmaceutically acceptable salts of the peptide identified by SEQ. ID. NO: 1, are new products and constitute a further aspect of this invention. The peptide identified by SEQ. ID. NO: 1 has been previously obtained by chemical synthesis and exhibited ACEI activity (Kohmura M, Nio N, Kubo K *et al.* 1989. Agric Biol. Chem 53:2107-2114). However, the obtainment thereof by proteolytic digestion of a substrate with *E. faecalis* has not been previously disclosed nor has the antihypertensive activity thereof been demonstrated. As is known, many peptides that appear as strong ACE inhibitors *in vitro* loose all or most of their ACEI activity when tested *in vivo* (Maruyama S, Mitachi H, Awaya J *et al.* 1987. Agric Biol Chem 51: 2557-2561; Maeno M, Yamamoto N, Takano T. 1996. J Dairy Sci 79: 1316-1321). On the other hand, and just as peptides with a strong inhibitory activity *in vitro* do not always act as antihypertensive agents, the opposite case may also occur, that is peptides which do not show great activity as ACE inhibitors *in vitro* acquire this ability *in vivo* due to the action of the digestive enzymes on them (Maeno M, Yamamoto N, Takano T. 1996. J Dairy Sci 79: 1316-1321). All these results show the need for proving *in vivo* the antihypertensive efficacy of peptides showing ACEI activity, initially on an animal model to finally confirm the results in humans (Darragh AJ. 2002. Bull IDF 375:25-31).

It has now been found that the bioactive peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7 and SEQ. ID. NO: 8, have ACEI activity and/or antihypertensive activity, and may therefore be used, particularly the antihypertensive peptides, in the elaboration of food or pharmaceutical compositions with ACEI activity and/or antihypertensive activity. Particularly the peptide identified by SEQ. ID. NO: 1 is an antihypertensive peptide showing a strong ACEI activity *in vitro,* as well as an antihypertensive activity tested in spontaneously hypertensive rats (SHR) and does not have antihypertensive activity tested in normotensive Wistar-Kyoto rats (WKY) when orally administered (Example 4). The fact that said peptide may reach its action site without being degraded, and consequently inactivated, by the digestive tract enzymes increases its application potential given that resistance to such enzymes is a prerequisite for a peptide to exert its effect *in vivo* after oral ingestion. Both the ACEI activity and the antihypertensive effect of said peptide (SEQ. ID. NO: 1) have been demonstrated with the pasteurized product, which indicates that the presence of live bacteria is not necessary for obtaining the ACEI activity and/or the antihypertensive effect tested in rats.

In another aspect, the invention refers to a composition comprising a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof, and mixtures thereof.

In another aspect, the invention provides a pharmaceutical composition comprising a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof, and mixtures thereof, and a pharmaceutically acceptable excipient. In a particular embodiment, said pharmaceutical composition is an antihypertensive pharmaceutical composition comprising at least one antihypertensive peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof, and mixtures thereof, and a pharmaceutically acceptable excipient. Said antihypertensive peptide is preferably the peptide identified by SEQ. ID. NO: 1 and/or a derivative thereof and/or a pharmaceutically acceptable salt thereof.

The pharmaceutical composition provided by this invention comprises a therapeutically effective amount of a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof and mixtures thereof. The amount of peptide, derivative or pharmaceutically acceptable salt thereof that may be present in the pharmaceutical composition provided by this invention may vary within a broad range, typically between 0.05% and 100% by weight, preferably between 0.16% and 99% by weight. Said pharmaceutical composition is useful for its administration and/or application in the human or animal body, preferably in the human being. The amount of peptide that must be administered, as well as its dosage, to treat a pathological condition will depend upon numerous factors, including age, the condition of the patient, the severity of the alteration or disorder, the route and frequency of administration and the specific peptide to be used.

The pharmaceutical compositions provided by this invention may be presented in any suitable administration form , solid or liquid, for example, and may be administered by any suitable route, for example orally, sublingually, by respiratory administration, parenterally, rectally or topically, for which the pharmaceutically acceptable excipients necessary for the formulation of the desired administration form will be included. In a particular embodiment, said pharmaceutical administration form of said peptides is a solid or liquid form designed for its oral administration. A review of the different pharmaceutical administration forms of drugs and of the excipients necessary for obtaining these can be found, for example, in the "Tratado de Farmacia Galénica" *[Galenic Pharmaceutical Treaty]*, C. Fauli i Trillo, 1993, Luzán 5, S.A. Ediciones, Madrid.

In another aspect the invention refers to the use of a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof, and mixtures thereof, in the elaboration of a drug for treating and/or preventing high blood pressure in all its forms in a human being or in an animal, and for treating and/or preventing any disorder associated with high blood pressure in a human being or in an animal, for example, ventricular hypertrophy, congestive heart failure, cerebrovascular accidents, retinopathy, progressive renal failure, dissecting aneurysms, coronary disease and peripheral vascular diseases.

The invention also provides a functional food product comprising a food product and a peptide chosen from the group formed by the peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof, and mixtures thereof. Said functional food product constitutes a further aspect of this invention. In a particular embodiment, said functional food product is a product with antihypertensive activity and is useful as a coadyuvant in treating and/or preventing high blood pressure in a mammal such as the human being, or conditions derived therefrom. Although practically any food product may be used in the elaboration of the functional food product of the invention, in a particular embodiment said food product comprises drinks, (for example soft drinks, carbonated drinks, etc.), infusion-type foods (for example vegetables, etc.), sauces, condiments, salad dressings, fruit juices, syrups, desserts (for example puddings, gelatins, fillings, baked products, frozen desserts such as ice-creams, sorbets, etc.), ice-creams, lightly frozen products, sweets (for example candies, etc.), chewing gum, etc. The amount of peptide or peptides present in the functional food product may vary within a broad range; however, in a particular embodiment the amount of peptide or peptides present in the functional food product is comprised between 0.001% and 1% by weight with respect to the total, preferably between 0.01% and 0.5% by weight.

The peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7, SEQ. ID. NO: 8, derivatives thereof, pharmaceutically acceptable salts thereof, and mixtures thereof, may also be ingested by human beings and animals as a food additive or supplement contained in foods and drinks. Said peptides may be formulated along with one or more nutritional substances, for example, vitamins and/or minerals, and incorporated in substantially liquid compositions such as nutritive drinks, milk, soy milk, soups, etc., or in substantially solid compositions, or in gelatins. Alternatively, said peptides may be prepared in powder form and incorporated as such in a food product. The amount of peptide provided by this invention that may be present in said foods or drinks may be similar to the dose contained in a typical pharmaceutical composition provided by this invention.

In another aspect, the invention provides a method for the treatment and/or prophylaxis of high blood pressure in a subject such as a human being or an animal, and for treating and/or preventing any disorder associated with high blood pressure in a human being or in an animal, such as ventricular hypertrophy, congestive heart failure, cerebrovascular accidents, retinopathy, progressive renal failure, dissecting aneurysms, coronary disease and peripheral vascular diseases, comprising the administration to said subject of an effective amount of an antihypertensive pharmaceutical composition provided by this invention in a amount suitable for producing an antihypertensive effect.

The peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7 and SEQ. ID. NO: 8 may be obtained by any suitable process, for example, by fermenting a substrate comprising one or more proteins, peptides or fragments thereof containing the amino acid sequence of said peptides with *E. faecalis,* for example, with *E. faecalis* CECT 5727, *E. faecalis* CECT 5728, *E. faecalis* CECT 5826 and/or *E. faecalis* CECT 5827 as previously mentioned, or rather by enzymatic digestion of said substrate using an enzyme with proteolytic activity. chemical peptide synthesis. Information on chemical peptide synthesis methods may be found, for example, in WO 01/68113. The derivatives and pharmaceutically acceptable salts of said peptides identified by SEQ. ID. NO: 1, SEQ. ID. NO: 2, SEQ. ID. NO: 3, SEQ. ID. NO: 4, SEQ. ID. NO: 5, SEQ. ID. NO: 6, SEQ. ID. NO: 7 and SEQ. ID. NO: 8 may be obtained by conventional methods, as previously mentioned.

In general, when obtaining an antihypertensive functional food product is desired, all necessary modifications in the previously described fermenting process will be performed in order to achieve a good ACEI activity in the fermented product, but controlling the production of bitterness, typically produced by the appearance of hydrophobic peptides of low molecular weight, the incidence of which is high after casein hydrolysis, due to the fact that their sequences contain a great number of hydrophobic amino acids. On the other hand, in the event that the antihypertensive peptides provided by this invention are used as ingredients of any type of food product in order to convert it into a functional food product with antihypertensive activity, obtaining fermented products with good organoleptic characteristics would lack importance given that the intention is to obtain the greatest possible amount of the antihypertensive peptide or the maximum ACEI activity in order to obtain it in a sufficient amount so as to use it as an ingredient. Finally, in the event of using one of said peptides, for example the peptide identified by SEQ. ID. NO: 1, as an antihypertensive drug the fermentation would be aimed towards obtaining the greatest possible concentration of the peptide in order to subsequently proceed to its isolation and purification by conventional methods, by high performance liquid chromatography (HPLC), for example.

Although the antihypertensive peptides provided by this invention do not generally show an ACEI activity as strong as that of the drug Captopril ($IC_{50}$ = 0.006 $\mu$M, being $IC_{50}$ the concentration of the inhibitor that reduces the enzymatic activity by 50%), these are natural peptides with which few side effects and good tolerance are expected. They would therefore be useful as coadjuvants in treating and/or preventing high blood pressure and consequently in the fight against cardiovascular disease.

The following examples illustrate the invention but should not be considered as limiting the scope thereof.

**EXAMPLE 1**

**Selection of strains of fermented milk producing microorganisms with ACEI activity: Isolation of *E. faecalis* CECT 5727, *E. faecalis* CECT 5728, *E. faecalis* CECT 5826 and**

**_E. faecalis_ CECT 5827**

1.1 Selection of fermented milk producing strains with high ACEI activity

231 microorganisms, isolated from raw bovine milk collected from different origins, were tested, checking their ability to produce fermented milks with ACEI activity by means of the process described below.

Aliquots of raw bovine milk of different origins were inoculated in dishes containing MRS medium (Oxoid Ltd, Basingstoke, UK) (selective for *Lactobacillus* genus) or M17 medium (Biokar Diagnostics, Beauvais, France)(selective for *Streptococcus* genus) and were incubated for 48 hours at 42°C in anaerobic atmosphere (AnaeroGen™, Oxoid Ltd, Basingstoke, UK), with less than 1% oxygen and a carbon dioxide level comprised between 9% and 13% (MRS medium), or at 30°C and in aerobic conditions (M17 medium). When on one same dish the growth of more than one different colony of microorganisms was observed, the isolation of the different colonies was performed by consecutive re-inoculations in the same mediums.

From all the organisms isolated, those with the ability to produce fermented milks with ACEI activity were chosen, using the protocol described in Example 2. At the end of fermentation, the fermented milks were centrifuged at 20,000 x g for 10 minutes to obtain the whey in which ACEI activity was tested. The enzymatic activity test was performed immediately, or otherwise the supernatants were kept frozen at -20°C.

The ACEI activity of the supernatants was determined by means of the test described in Example 1.2. The bacteria the supernatant of which did not show ACEI activities or ACEI activities under 70% when the supernatant diluted to half was tested were discarded. A total of 47 bacteria were chosen that were identified by their fermenting and biochemical profile by means of API strips (BioMérieux SA, Marcy l'Etoile, France), and incorporated to the Grupo Leche Pascual, S.A. Culture Collection (Table 1). The most promising strains were deposited at the Spanish Type Culture Collection (CECT) Burjassot, Valencia, on October 10th, 2002 and July 17th, 2003, and among these the strains of *Enterococcus faecalis* identified with access numbers CECT 5727, CECT 5728, CECT 5826 and CECT 5827. These strains can grow on M17 medium in aerobiosis at 30°C.

The results obtained with the bacteria chosen due to their ability to produce fermented milk with ACEI activity exceeding 70% measured in the supernatant diluted to half and incorporated to the Grupo Leche Pascual, S.A. Culture Collection are included in Table 1. In most cases, two values of ACEI activity are shown due to the fact that repetitions were made to confirm the results. The last column refers to the amount of supernatant necessary to produce an enzymatic reduction of 50% ($IC_{50}$) expressed in micrograms/mL. The Kjeldhal method (Norm FIL-IDF 20B 1993) was used in order to know the peptide concentration in the fermented milks.

**Table 1**

| Microorganism | ACEI activity | | $IC_{50}$ ($\mu$g/mL) |
|---|---|---|---|
| **_Enterococcus faecalis_ (CECT 5727)** | **100 %** | **100 %** | **39** |
| **_Enterococcus faecalis_ (CECT 5728)** | **99.0 %** | **100 %** | **59** |
| **_Enterococcus faecalis_ (CECT 5826)** | **100 %** | **96.4 %** | **49** |
| **_Enterococcus faecalis_ (CECT 5827)** | **100 %** | **99.0 %** | **34** |
| *Lactococcus lactis cremoris* | 92.3 % | 87.8 % | 520 |
| *Lactococcus lactis cremoris* | 86.2 % | 92.6 % | |
| *Lactococcus lactis cremoris* | 99.8 % | 83.0 % | |
| *Lactobacillus helveticus* | 77.9 % | 64.6 % | 95 |

Table continued

| Microorganism | ACEI activity | | IC$_{50}$ (μg/mL) |
|---|---|---|---|
| *Lactobacillus helveticus* | 86.6 % | 83.3 % | |
| *Lactobacillus helveticus* | 87.4 % | | |
| *Lactobacillus brevis* | 91.7 % | 85.8 % | |
| *Lactobacillus fermentum* | 100 % | 100 % | |
| *Lactobacillus fermentum* | 92.1 % | 61.7% | |
| *Lactobacillus rhamnosus* | 81.3 % | 78.2% | 194 |
| *Lactobacillus rhamnosus* | 80.1 % | 76.3% | 202 |
| *Lactobacillus rhamnosus* | 83.6 % | 76.5% | 175 |
| *Lactobacillus rhamnosus* | 74.5% | 74.9% | |
| *Lactobacillus rhamnosus* | 72.3 % | 73.2 % | |
| *Lactobacillus rhamnosus* | 91.0 % | 97.2 % | |
| *Lactobacillus rhamnosus* | 97.7 % | 71.2 % | |
| *Lactobacillus rhamnosus* | 84.0 % | 94.2 % | |
| *Lactobacillus rhamnosus* | 85.5 % | 71.1 % | |
| *Lactobacillus rhamnosus* | 84.0 % | 76.8 % | |
| *Lactobacillus rhamnosus* | 100 % | 98.1 % | |
| *Lactobacillus rhamnosus* | 75.6 % | 76.0 % | |
| *Lactobacillus rhamnosus* | 74.3% | 96.3% | |
| *Lactobacillus rhamnosus* | 87.8 % | 68.2 % | |
| *Lactobacillus rhamnosus* | 84.2 % | 86.8% | |
| *Lactobacillus rhamnosus* | 90.6 % | 92.2 % | |
| *Lactobacillus rhamnosus* | 81.8 % | 89.4 % | |
| *Lactobacillus rhamnosus* | 91.9 % | 100 % | |
| *Lactobacillus rhamnosus* | 77.1 % | 81.6 % | |
| *Lactobacillus rhamnosus* | 97.1 % | 89.7 % | |
| *Lactobacillus rhamnosus* | 79.7 % | 97.9 % | |
| *Lactobacillus paracasei paracasei* | 91.8 % | | |
| *Lactobacillus paracasei paracasei* | 99.6 % | 99.8 % | |
| *Lactobacillus para paracasei* | 80.0 % | 37.4 % | |
| *Lactobacillus acidophilus* | 75.0 % | 67.8 % | 88 |
| *Lactobacillus acidophilus* | 92.0 % | 96.8 % | 89 |
| *Lactobacillus acidophilus* | 89.6 % | 90.4 % | 383 |
| *Lactobacillus acidophilus* | 75.3 % | 92.2 % | 389 |
| *Lactobacillus acidophilus* | 91.1 % | 95.2 % | 178 |
| *Lactobacillus acidophilus* | 85.0 % | 93.8 % | |
| *Lactobacillus acidophilus* | 89.4 % | 87.7 % | |
| *Lactobacillus acidophilus* | 95.4 % | 95.7 % | |
| *Lactobacillus acidophilus* | 86.8 % | 88.0 % | |
| Unidentifiable strain | 75.3 % | 92.6 % | |

1.2 Measurement of ACE Inhibitor activity (ACEI activity)

The measurement of ACE activity was performed according to the method disclosed by Cushman and Cheung (Cushman DW, Cheung HS. 1971. Biochem Pharmacol 20:1637-1648) and subsequently modified by Nakamura (Nakamura Y, Yamamoto N, Sakai K *et al.* 1995. J Dairy Sci 78:777-783). This method is based on the use of Hippuryl-L-Histidyl-L-Leucine (Hip-His-Leu) as substrate given that the action of the ACE on such substrate produced the release of hippuric acid, easily quantifiable by spectrophotometry by absorbance reading at 228 nm (A$_{228}$) after extraction thereof with ethyl acetate. If the sample to be tested is added to the reaction tube along with the ACE and the substrate, the ACEI activity of the sample tested can be determined. As is mentioned in Example 1.1, during the selection of bacteria the sample used to measure ACEI activity was the supernatant of the fermented milk, subjected to centrifugation at 20,000 x g for 10 minutes and subsequently diluted to half with distilled water.

The test is performed by pipetting the reagents indicated in Table 2 in Eppendorf tubes.

**Table 2**

|  | Sample (μL) (A) | Sample Blank (μL) (B) | Enzyme (μL) (C) | Substrate Blank (μL) (D) |
|---|---|---|---|---|
| Supernatant | 40 | 40 | 0 | 0 |
| Substrate solution | 100 | 0 | 100 | 100 |
| ACE Enzyme Solution (0.1 U/mL) | 20 | 0 | 20 | 0 |
| H$_2$O | 0 | 120 | 40 | 60 |

The substrate solution was formed by 0.1 M sodium borate, 300 mM sodium chloride, 5 mM Hip-His-Leu (Sigma, St. Louis, MO), pH 8.3 at 37°C. The ACE enzyme solution (Sigma, St. Louis, MO) was adjusted to an activity of 0.1 U/mL.

The different reaction mixtures were incubated for 30 minutes at 37°C and subsequently 150 μL of 1 M hydrochloric acid were added in order to stop the reaction and subsequently 1,000 μL, of ethyl acetate in order to extract the hippuric acid. The resulting mixtures were vigorously stirred for 20 seconds and centrifuged at 1,500 x g for 10 minutes in order to separate and collect from each tube the upper phase (ethyl acetate). 750 μL of each upper phase were pipetted into individual tubes, which were introduced in a bath of boiling water under a fume extraction hood until the solvent evaporated. Once the ethyl acetate was eliminated 800 μL of distilled water were added to each tube to dissolve the dry residue smoothly and without stirring. Finally the content of each tube was transferred to quartz cuvettes to read their absorbance at 228 nm. The inhibition percentage (% Inhibition) was calculated by applying the following formula:

$$\% \text{ Inhibition} = \frac{(A) - (B)}{(C) - (D)}$$

wherein (A), (B), (C) and (D) correspond to the absorbances at 228 nm of the solutions indicated in Table 2.

**EXAMPLE 2**

**Production of fermented milk with ACEI activity by the use of *E. faecalis* CECT 5727, *E. faecalis* CECT 5728, *E. faecalis* CECT 5826 and/or *E. faecalis* CECT 5827.**

To perform this fermentation example, *E. faecalis* CECT 5727, *E. faecalis* CECT 5728, *E. faecalis* CECT 5826 or *E faecalis* CECT 5827 may be used indistinctly. The substrate is skimmed bovine milk powder reconstituted in water at 10% and autoclaved (100°C for 20 minutes). First a starter liquid was made, inoculating the substrate with a culture loop such that the initial population was $10^5$-$10^7$ CFU/mL and incubating for 24 hours. 3% by weight of this culture was used as inoculum for the second fermentation which was maintained for 48 hours. Both fermentations were carried out at 30°C. The fermenting process was stopped by pasteurizing the fermented milk [75°C for 1 minute] and the final pasteurized product was used to measure ACEI activity according to the protocol described in Example 1.2, although in this case the fermented milks were subjected to the pasteurization treatment mentioned before being centrifuged at 20,000 x g for 10 minutes. Figure 1 shows the ACEI activity test for different supernatant volumes, suitably diluted with distilled water in order to keep a constant reaction volume (in Example 1 the dilution was to half). Therefore, the final reaction volume is always 160 μL [100 μL of the substrate solution, 20 μL of the ACE solution and 40 μL of the sample to be tested (supernatant at the corresponding dilution with distilled water)]. The results found show that fermented milks produced by both microorganisms show a strong ACEI activity, given that with small sample volumes inhibitions of 100% of the activity are obtained.

Additionally, the occurrence over time of ACEI activity was studied in milks fermented with strains of *E. faecalis* CECT 5727, *E. faecalis* CECT 5728, *E. faecalis* CECT 5826 and/or *E. faecalis* CECT 5827, as well as the degree of hydrolysis of said strains. Fermentations were carried out according to the protocol described in this very Example 2, but extracting aliquots of the second fermentation at different times: 8, 12, 24 and 48 hours.

The ACEI activity was determined by the previously described protocol and is represented as the activity of the whey dilution that produces a 50% inhibition of the activity of the enzyme expressed in inhibition units per volume (IU/mL), understanding as an inhibition unit that capable of inhibiting 50% of the activity of the ACE enzyme.

For the study of the degree of hydrolysis a quantitative analysis of the peptides was performed according to the OPA method (Church FC, Swaisgood HE, Porter DH., Catigna

GL. 1983. J Dairy Sci 66: 1219-1227). Casein peptone was used for the calibration curve (Fluka, Steinheim, Switzerland).

The results showed that the ACEI activity and the proteolytic activity, measured by OPA, follow a typical primary metabolite pattern, with a pronounced initial increase associated to the exponential growth of the culture, until reaching a final plateau (Figure 2).

**EXAMPLE 3**

**Identification, isolation and synthesis of peptides with ACEI activity**

In order to carry out this work, analytical and preparative high performance liquid chromatography (HPLC) equipment was used, as well as mass spectrometry (MS) equipment in tandem which allowed sequencing of the peptides. The following steps were performed.

3.A Obtaining the soluble fraction of the fermented dairy products

Dairy products fermented by strains of *E. faecalis* CECT 5727, *E. faecalis* CECT 5728, *E. faecalis* CECT 5826 and/or *E. faecalis* CECT 5827 (separately) were obtained following the process described in Example 2. Briefly, bovine milk powder reconstituted in water at 10% and autoclaved (100°C for 20 minutes) was subjected to a first 24 hour fermentation with an approximate amount of bacteria of $10^7$-$10^9$ for every 100 mL of reconstituted milk, and a second 48 hour fermentation with an inoculum of the first fermentation of 3% by weight, both fermentations carried out at 30°C and stopping the fermenting process by pasteurization of the fermented milk at 75°C for 1 minute. The resulting products were centrifuged at 20,000 x g for 10 minutes. The supernatants obtained where ultrafiltered through a hydrophilic membrane of 3,000 Da pore size (Centripep, Amicon Inc, Beverly, MA, USA). The permeates obtained were frozen, lyophilized and kept at -20°C until later fractioning thereof.

3.B Fractioning by reversed-phase HPLC at semipreparative scale

The equipment used (Waters Series 600 HPLC; Waters Corp., Mildford, MA, USA) has a pump (delta 600 model), a gradient controller (600 model), an injector (717 plus model), an ultraviolet detector of variable wavelength (996 model), a fraction collector and data acquisition and processing software (Millenium 3.2). The column is a Prep Nova Pak® HR $C_{18}$ (300 x 7.8 mm ID, 6 $\mu$m particle size, 60 Å pore size)(Waters). Solvent A is a mixture of water and trifluoroacetic acid (1000:1) and solvent B is a mixture of acetonitrile and trifluoroacetic acid (1000:0.8). The mixtures were eluted with a 4 mL/minute flow with a linear gradient of 0% to 35% of solvent B in A in 70 minutes. The absorbance of the solvent was monitored at 214 nm and 280 nm.

Briefly, each lyophilized permeate [obtained en 3.A] was dissolved in water at a concentration of 100 mg/mL and at each separation, the injected sample volume was 500 $\mu$L. The fractions that were separated were tested in order to determine the existence of ACEI activity, according to the protocol described in Example 1.2, observing that peptides with ACEI activity eluted at the end of the chromatogram with retention times exceeding 47 minutes. As an example, Figure 3 shows the chromatogram corresponding to the permeate of the milk fermented by the strain of *E. faecalis* CECT 5727. This fraction was subjected to a second analysis by reversed-phase HPLC at preparative scale using the same equipment, column and solvents but eluting the sample with a linear gradient from 20% to 35% of solvent B in A in 40 minutes (Figure 4). In those conditions, ACEI activity was observed in fractions that eluted between minutes 12 and 42 (Figure 4, fractions 1 to 7). Said fractions, which contained peptides with ACEI activity, were collected, lyophilized and kept at -20°C until their subsequent identification.

3.C Identification of peptides with ACEI activity by means of mass spectrometry in tandem (MS/MS)

Analysis by mass spectrometry was performed in an Esquire3000 (Bruker Daltonik GmbH, Bremen, Germany) ion trap mass analyzer. The peptides collected from HPLC and lyophilized [from 3.B] were dissolved at a concentration of 5-10 $\mu$g/ml in a mixture of 50% acetonitrile in water containing 0.3% formic acid. The sample was injected in the electrospray sprayer at a flow of 4 $\mu$L/min using a type 22 syringe pump (Harvard Apparatus, South Natick, MA, USA). The analyzer uses nitrogen as a sprayer and drying gas and operates with a helium pressure of 5 x $10^{-3}$ bar. The mass

spectrums are obtained in 50-1500 *m*/*z* intervals at a speed of 13,000 Da/second. Interpretation of the tandem MS spectrums for identifying the peptide sequences was performed with the Biotools 2.1 program (Bruker Daltonik GmbH, Bremen, Germany). The peptides included in Table 3 were identified.

**Table 3 Peptides identified in bovine milk fermented with CECT 5727, with CECT 5728, with CECT 5826 and/or with CECT 5827**

| Peptide | SEQ. ID. NO: | Theoretical mass[a] | Experimental mass | Sequence |
|---|---|---|---|---|
| LHLPLP | 1 | 688.87 | 688.4 | 133-138 β-bovine casein |
| VLGPVRGPFP | 2 | 1.038.26 | 1.037.6 | 197-206 β-bovine casein |
| LHLPLPL | 3 | 802.03 | 801.5 | 133-139 β-bovine casein |
| VRGPFPIIV | 4 | 997.25 | 996.6 | 201-209 β-bovine casein |
| VVVPPF | 5 | 656.82 | 656.3 | 82-87 β-bovine casein |
| LTQTPVVVPPF | 6 | 1.197.44 | 1196.6 | 77-87 β-bovine casein |
| VLGPVRGPFPIIV | 7 | 1.363.71 | 1362.7 | 197-209 β-bovine casein |
| LVYPFPGPIPNSLPQNIPP | 8 | 2060.42 | 2059.4 | 58-76 β-bovine casein A[2] |
| [a] Mean value | | | | |

3.D Synthesis of the peptides with ACEI activity

Subsequently, the previously identified peptides with ACEI activity were chemically synthesized. The synthesis of said peptides with ACEI activity was performed by the Fmoc method in solid phase with a synthesizer (model 431A; Applied Biosystems Inc., Überlingen, Germany). The purity of the synthetic peptides was verified by reversed-phase HPLC at analytic scale and mass spectrometry using an Esquire-LC (Bruker Daltonik GmbH, Bremen, Germany) apparatus. The apparatus (1100 series) had a quaternary pump, an automatic mass injector, an eluent degassing system and a variable wavelength ultraviolet detector (Agilent Technologies, Waldbronn, Germany) coupled in line with an Esquire 3000 (Bruker Daltonik) ion trap mass spectrometer. The column was a Hi-Pore $C_{18}$ (250 x 4.6 mm ID, 5 $\mu$m particle size) column (Bio-Rad Laboratories, Richmond, CA, USA). Solvent A was a mixture of water and trifluoroacetic acid (1000:0.37) and solvent B a mixture of acetonitrile and trifluoroacetic acid (1000:0.27). The samples were eluted with 0.8 mL/minute flow with a linear gradient of 0% to 30% of solvent B in A in 25 minutes. The eluent was monitored at 214 nm, by means of mass spectrometry, under the same conditions as those indicated in 3.C, except that the injection flow of the sample through the sprayer was of 60 $\mu$L/min.

In the milks fermented with *E. faecalis* CECT 5727, with *E. faecalis* CECT 5728, with *E. faecalis* CECT 5826 and/or with *E. faecalis* CECT 5827 and in the synthesized peptides (which corresponded to the peptides isolated in the fermented milk) the concentration necessary to produce a reduction of 50% of the enzyme activity of ACE ($IC_{50}$, expressed en $\mu$M in the case of the peptides and in $\mu$g/mL in the case of the fermented milks) was determined. The values obtained are included in Table 4.

**Table 4 $IC_{50}$ values for peptides isolated from the milk fermented by *E. faecalis* CECT 5727, by *E. faecalis* CECT 5728, by *E. faecalis* CECT 5826 and/or by *E. faecalis* CECT 5827**

| Sample | $IC_{50}$ |
|---|---|
| Milk fermented by *E. faecalis* CECT 5727 | 39 $\mu$g/mL |
| Milk fermented by *E. faecalis* CECT 5728 | 59 $\mu$g/mL |
| Milk fermented by *E. faecalis* CECT 5826 | 49 $\mu$g/mL |

Table continued

| Sample | IC$_{50}$ |
|---|---|
| Milk fermented by *E faecalis* CECT 5827 | 34 μg/mL |
| SEQ. ID. NO: 1 | 5.4 μM |
| SEQ. ID. NO: 2 | 137.24 μM |
| SEQ. ID. NO: 3 | 432.7 μM |
| SEQ. ID. NO: 4 | 630.0 μM |
| SEQ. ID. NO: 5 | >700 μM |
| SEQ. ID. NO: 6 | >700 μM |
| SEQ. ID. NO: 7 | >700 μM |
| SEQ. ID. NO: 8 | 5.3 μM |

The first four peptides were initially chosen in order to test the antihypertensive activity thereof in spontaneously hypertensive rats (SHR) and in Wistar-Kyoto rats (WKY).

**EXAMPLE 4**

**Study of the antihypertensive activity of milks fermented with *E. faecalis* CECT 5727, with *E. faecalis* CECT 5728, with *E. faecalis* CECT 5826 and/or with *E. faecalis* CECT 5827 and peptides with ACEI activity identified in said milks**

Given the high ACEI activity shown in bovine milk fermented with *E. faecalis* CECT 5727, with *E. faecalis* CECT 5728, *E. faecalis* CECT 5826 and with *E. faecalis* CECT 5827 as well as some of the identified peptides, particularly the peptide identified by SEQ. ID. NO: 1, the effect of said fermented milks and of said peptides on the blood pressure of spontaneously hypertensive rats (SHR) and of Wistar-Kyoto rats (WKY), which are the normotensive control of SHR rats was studied.

The fermented milks were prepared according to the process described in Example 2. The tested peptides were chemically synthesized following the protocol described in Example 3.D.

Male SHR and WKY rats of 17-20 weeks of age and weight comprised between 250 and 300 g were used, obtained from Charles River Laboratories Espana S.A.. The rats were kept at a stable ambient temperature of 23°C and with 12 hour light-dark cycles, taking water and food at will. The measurement of blood pressure in these animals was carried out by means of a modification of the tail cuff technique, originally disclosed by Buñag (Buñag RD. 1973. J Appl Physiol 34:79). This technique usually allows obtaining only systolic blood pressure (SBP) measurements; however, the equipment used in this study (Le50001 Letica model) allows differentiating the SBP and the diastolic blood pressure (DBP) of the animals, as it automatically provides the digital value of both parameters. For this reason the modification of the SBP and the DBP produced by the acute administration of the fermented milks and the peptides were tested in this study. Before placing the cuff on the tails of the rats, these were exposed to a temperature close to 30°C in order to facilitate the dilation of the caudal artery, and they were lightly anaesthetized with diethyl ether according to the process disclosed by Dietz et al. (Dietz R, Schömig A, Haebara H *et al.* 1978. Circ Res 43: I98-I106) in order to minimize the effect of stress on the measurement. Furthermore, in order to ensure the reliability of the measurement, the animals were familiarized with the process 2 weeks prior to performing the test in question.

The administration of the products to be tested was performed by means of intragastric probe in a time margin comprised between 9 and 10 am. The SHR rats used for the study had at that time SBP and DBP values comprised respectively between 240 and 290 mm Hg, and between 200 and 250 mm Hg. The WKY rats used for the study had at that time SBP and DBP values comprised respectively between 160 and 200 mm Hg, and between 100 and 140 mm Hg. SBP and DBP measurements were taken periodically in the animals, every 2 hours, up to 8 hours post-administration of the products to be tested; an additional measurement of both variables (SBP and DBP) was taken 24 hours after administration of said products.

In the case of the fermented milks, the supernatant (whey) obtained by centrifugation at 20,000 x g for 10 minutes of the milk fermented and pasteurized at 75°C for 1 minute following the process described in Example 2 was used as an administration product. From the corresponding supernatant a single administration of 5 mL/kg of animal body weight was used.

In the tests with peptide SEQ. ID. NO: 1 a single administration was also performed, and the dose used (2 mg/kg) was equivalent in ACEI activity units to the 5 mL/kg dose of supernatant of the milks fermented by *E. faecalis* CECT 5727, *E. faecalis* CECT 5728, *E. faecalis* CECT 5826 and *E. faecalis* CECT 5827. The peptides were always dissolved in distilled water, and the corresponding dose was always administered to each rat in a 1 mL volume, given that said volume was comparable to the volume of fermented milk that each animal received. The most significant results were obtained with the peptide identified by SEQ. ID. NO: 1.

As a negative control (to establish the circadian variation of the SBP and the DBP in probed rats) the measurements obtained in rats that were administered 1 mL of water by intragastric probe were used. As a positive control (to establish the effect on the SBP and the DBP of a prototype ACEI drug, such as Captopril) the measurements of SBP and SBP obtained in that were administered 50 mg /kg of Captopril by intragastric probe were used. This dose of Captopril was administered to each rat in a volume of 1 mL.

Likewise, tests similar to those previously described were carried out in which the animals were treated by the same process with 1 mL of bovine milk (control). Said tests allowed establishing the effect of non-fermented milk on the blood pressure of the animals. The results of these tests are not represented in the figures because the corresponding SBP and DBP values were similar to the values of SBP and DBP of the animals to which water was administered.

The results obtained were grouped and the mean $\pm$ standard error of the mean (SEM) for a minimum of 7 homogeneous tests was obtained. The data from treated animals was contrasted to one another and with the data considered as negative control. In order to compare them and obtain the statistical significance the one-way analysis of variance was performed (ANOVA), the differences between the different treatments for each time studied being studied by means of the LSD (Least Significant Difference) test, being considered significant the difference for values of p<0.05 (Tables 5A and 5B).

Figures 5A and 5B show, respectively, the decrease in SBP and DBP obtained in SHR rats at different times after administration of the milks fermented with the chosen strains of *E. faecalis* (CECT 5727, CECT 5728, CECT 5826 and CECT 5827). Said figures also include the reduction in SBP and in DBP observed after administration of Captopril. As could be predicted, Captopril produced a pronounced reduction in SBP and DBP in SHR rats. The reduction of the SBP was maximum 6 hours after administration of the drug, while the reduction of DBP was maximum 4 hours after administration thereof. The wheys of the fermented milks caused a reduction in SBP and DBP in the animals that was less than the reduction produced by Captopril, but that was also significant. The smaller values of SBP after administering the milks fermented with *E. faecalis* (CECT 5727, CECT 5728, CECT 5826 and CECT 5827) were observed between 4 and 6 hours after administration thereof. At that moment very low values of DBP were also obtained, and the values of this variable remained low and practically without variation up to 6 hours after administration. The values of SBP and DBP observed 24 hours after the different administrations were similar to those the animals had before these (Tables 5A and 5B).

**Table 5A Mean values $\pm$ standard error of the variation of systolic blood pressure in SHR rats at different times after administration of water, Captopril and milks fermented with *E. faecalis* CECT 5727, *E. faecalis* CECT 5728, *E. faecalis* CECT 5826 and *E. faecalis* CECT 5827**

|  | Time 2 h | Time 4 h | Time 6 h | Time 8 h | Time 24 h |
|---|---|---|---|---|---|
| Water | -10.69±4.70[c] | -11.79±8.28[c] | -13.73±7.25[c] | -8.08±7.02[c] | 3.92±3.52[a] |
| CECT 5727 | -32.98±5.43[ab] | -34.81±4.48[b] | -28.79±4.45[b] | -14.81±2.82[bc] | 4.46±1.21[a] |
| CECT 5728 | -24.62±4.58[abc] | -34.81±4.84[b] | -34.76±4.05[b] | -18.43±4.05[bc] | -1.86±1.41[a] |
| CECT 5826 | -27.67±3.79[ab] | -32.62±4.10[b] | -28.96±4.56[b] | -21.42±3.58[b] | 0.46±1.47[a] |
| CECT 5827 | -20.13±6.24[bc] | -30.79±3.91[b] | -26.92±4.20[bc] | -13.71±1.89[bc] | -1.82±4.03[a] |
| Captopril | -36.08±4.52[a] | -51.98±5.36[a] | -54.19±5.11[a] | -35.44±4.70[a] | 1.23±1.49[a] |

[a,b,c,d]The same letter in the same column indicates non-significant differences between mean values (*P*<0.05).

**Table 5B Mean values $\pm$ standard error of the variation of diastolic blood pressure in SHR rats at different times after administration of water, Captopril and milks fermented with *E. faecalis* CECT 5727, *E. faecalis* CECT 5728, *E. faecalis* CECT 5826 and *E. faecalis* CECT 5827**

|  | Time 2 h | Time 4 h | Time 6 h | Time 8 h | Time 24 h |
|---|---|---|---|---|---|
| Water | -3.19±2.78[d] | -4.29±3.00[b] | -8.62±3.30[b] | -5.00±2.42[b] | -0.88±1.04[a] |
| CECT 5727 | -24.06±5.59[ab] | -25.50±6.18[a] | -27.36±7.21[a] | -18.92±5.37[a] | -0.04±1.04[a] |
| CECT 5728 | -16.79±3.56[bc] | -27.38±3.35[a] | -24.04±3.24[a] | -16.98±2.46[ab] | 0.27±1.33[a] |
| CECT 5826 | -7.87±3.86[cd] | -24.08±4.70[a] | -27.73±3.93[a] | -20.02±4.29[a] | 1.17±0.62[a] |

Table continued

| | Time 2 h | Time 4 h | Time 6 h | Time 8 h | Time 24 h |
|---|---|---|---|---|---|
| CECT 5827 | -15,10±4.02[bc] | -30.49±5.14[a] | -24.73±4.22[a] | -16.10±1.39[ab] | 1.66±1.32[a] |
| Captopril | -28.29±2.40[a] | -34.90±5.34[a] | -28.63±5.77[a] | -26.67±7.94[a] | 1.37±1.02[a] |

[a,b,c] The same letter in the same column indicates non-significant differences between mean values ($P < 0.05$).

Figures 6A and 6B show, respectively, the changes in SBP and DBP obtained in WKY rats at different times after administration of the wheys fermented with *E. faecalis* (CECT 5727, CECT 5728, CECT 5826 and CECT 5827). Also included are the results obtained after administration of Captopril. It can be observed that neither the fermented milks nor Captopril modified the SBP or the DBP of the animals treated; no significant differences being observed when compared for each time studied by means of the LSD test. These results allow discarding any possible side effects of the fermented products tested on the blood pressure of normotensive subjects.

Figures 7A and 7B show, respectively, the changes in the reduction in SBP and in DBP obtained in SHR rats at different times, after administration of peptide SEQ. ID. NO: 1, at a dose of 2 mg/kg of body weight. It can be observed that the administration of said dose of this peptide causes a significant reduction in the SBP in these animals. The reduction in SBP was already acute 2 hours after administration, the smallest values of SBP being obtained 4 hours after administration of the peptide. The peptide identified by SEQ. ID. NO: 1 was capable of reducing the DBP of SHR rats even more than the SBP of these animals. Furthermore, its effect on the DBP of the SHR rats was very fast, and 2 hours after administration thereof the smallest values of this variable (DBP) were observed. It is worth mentioning that the maximum reduction of SBP and SBP produced by the peptide were even greater than the maximum reduction of these variables produced by the tested fermented milks (represented in Figures 5A and 5B). However, 8 hours after administration of the peptide the rats had recovered values of SBP and DBP similar to those these animals had before administration thereof. These results demonstrate that the peptide identified by SEQ. ID. NO: 1 has a clear and pronounced antihypertensive effect which, after acute administration thereof, appears and disappears before the antihypertensive effect of the fermented milks containing it.

**Claims**

1. A strain of *Enterococcus faecalis* deposited at the CECT chosen from the strain identified as *E. faecalis* CECT 5727, the strain identified as *E. faecalis* CECT 5728, the strain identified as *E. faecalis* CECT 5826 and the strain identified as *E. faecalis* CECT 5827.

2. A bacterial preparation containing a strain of *E. faecalis* chosen from *E. faecalis* CECT 5727, *E. faecalis* CECT 5728, *E. faecalis* CECT 5826, *E. faecalis* CECT 5827 and mixtures thereof.

3. A bacterial preparation according to claim 2, further containing one or more different microorganisms.

4. A. bacterial preparation according to any of claims 2 or 3, in the form of lyophilized powder or in a capsule.

5. Use of a strain of *E. faecalis* chosen from *E. faecalis* CECT 5727, *E. faecalis* CECT 5728, *E. faecalis* CECT 5826, *E. faecalis* CECT 5827 and mixtures thereof in the food industry.

6. A food product comprising a strain of *Enterococcus faecalis* according to claim 1, or a bacterial preparation according to any of claims 2 to 4, or prepared by using said strain or bacterial preparation.

7. A food product according to claim 6, chosen from a dairy product, optionally fermented and/or subjected to heat treatment, and a drink.

8. A food product according to claim 7, chosen from a fermented and pasteurized dairy product, a drink containing whey, a fruit drink and a beer.

9. Use of a strain of *E. faecalis* chosen from *E. faecalis* CECT 5727, *E. faecalis* CECT 5728, *E. faecalis* CECT 5826, *E. faecalis* CECT 5827 and mixtures thereof in the elaboration of a drug for treating and/or preventing high blood pressure.

10. Use of a strain of *E. faecalis* chosen from *E. faecalis* CECT 5727, *E. faecalis* CECT 5728, *E. faecalis* CECT 5826, *E. faecalis* CECT 5827 and mixtures thereof in the elaboration of a drug for treating and/or preventing high blood pressure in all its forms in a human being or in an animal, and for treating and/or preventing any disorder associated with high blood pressure in a human being or in an animal.

11. Use of a strain of *E. faecalis* chosen from *E. faecalis* CECT 5727, *E. faecalis* CECT 5728, *E. faecalis* CECT 5826, *E. faecalis* CECT 5827 and mixtures thereof in the elaboration of a drug for treating disorders associated with high blood pressure chosen from ventricular hypertrophy, congestive heart failure, cerebrovascular accidents, retinopathy, progressive renal failure, dissecting aneurysms, coronary disease and peripheral vascular diseases.

12. Use of a strain of *E. faecalis* chosen from *E. faecalis* CECT 5727, *E. faecalis* CECT 5728, *E. faecalis* CECT 5826, *E. faecalis* CECT 5827 and mixtures thereof in obtaining bioactive peptides.

13. Use of a strain of *E. faecalis* chosen from *E. faecalis* CECT 5727, *E. faecalis* CECT 5728, *E. faecalis* CECT *5826, E. faecalis* CECT 5827 and mixtures thereof in obtaining peptides with angiotensin-converting enzyme inhibiting activity and/or with antihypertensive activity.

14. Use according to claims 12 or 13, wherein said peptides are chosen from the group formed by the peptides identified by SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, derivatives thereof, pharmaceutically acceptable salts thereof and mixtures thereof.

15. A process for producing a bioactive peptide comprising fermenting a substrate comprising one or more proteins, peptides, or fragments thereof containing the amino acid sequence of said bioactive peptide and/or converting it into a derivative and/or into a pharmaceutically suitable salt.

16. A process according to claim 15, wherein said bioactive peptide is a peptide with ACEI activity and/or with antihypertensive activity.

17. A process according to claim 16, wherein said bioactive peptide is chosen from the group formed by the peptides identified by SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, derivatives thereof, pharmaceutically acceptable salts thereof and mixtures thereof.

18. A process according to claim 14, wherein said strain of *E. faecalis* is chosen from the group formed by *E. faecalis* CECT 5727, *E. faecalis* CECT 5728, *E. faecalis* CECT 5826, *E. faecalis* CECT 5827 and mixtures thereof.

19. A process according to claim 15, wherein said substrate to be fermented is a source of proteins, peptides or fragments thereof, comprising one or more proteins, peptides or fragments thereof containing the amino acid sequence of said bioactive peptide.

20. A process according to claim 19, wherein said substrate is of animal or plant origin, or comes from a microorganism.

21. A process according to claim 20, wherein said substrate comprises milk or a dairy product, optionally fermented, containing one or more milk proteins or a soy protein extract.

22. A process according to claim 21, wherein said substrate comprises milk or a dairy product, optionally fermented, containing casein.

23. A peptide chosen from the group formed by the peptides identified by SEQ. ID. NO.: 2, SEQ. ID. NO.: 3, SEQ. ID. NO.: 4, SEQ. ID. NO.: 6, SEQ. ID. NO.: 7, the derivatives thereof and pharmaceutically acceptable salts thereof, and by the derivatives and pharmaceutically acceptable salts of the peptide identified by SEQ. ID. NO.: 1.

**24.** A composition comprising a peptide chosen from the group formed by the peptides identified by SEQ. ID. NO.: 2, SEQ. ID. NO.: 3, SEQ. ID. NO.: 4, SEQ. ID. NO.: 6, SEQ. ID. NO.: 7, derivatives thereof, pharmaceutically acceptable salts thereof and mixtures thereof.

**25.** A pharmaceutical composition comprising a peptide chosen from the group formed by the peptides identified by SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, derivatives thereof, pharmaceutically acceptable salts thereof, and mixtures thereof, and a pharmaceutically acceptable excipient.

**26.** An antihypertensive pharmaceutical composition comprising at least one antihypertensive peptide chosen from the group formed by the peptides identified by SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, derivatives thereof, pharmaceutically acceptable salts thereof and mixtures thereof, and a pharmaceutically acceptable excipient.

**27.** A composition according to any of claims 25 or 26, wherein said peptide is the peptide identified by SEQ. ID. NO.: 1.

**28.** Use of a peptide chosen from the group formed by the peptides identified by SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, derivatives thereof, pharmaceutically acceptable salts thereof and mixtures thereof in the elaboration of a drug for treating and/or preventing high blood pressure in all its forms in a human being or in an animal, and for treating and/or preventing any disorder associated with high blood pressure in a human being or in an animal.

**29.** Use of a peptide chosen from the group formed by the peptides identified by SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, derivatives thereof, pharmaceutically acceptable salts thereof and mixtures thereof in the elaboration of a drug for treating and/or preventing in a human being or in an animal a disorder associated with high blood pressure chosen from ventricular hypertrophy, congestive heart failure, cerebrovascular accidents, retinopathy, progressive renal failure, dissecting aneurysms, coronary disease and peripheral vascular diseases.

**30.** Use according to any of claims 28 or 29, wherein said drug is administrated orally.

**31.** A functional food product comprising a food product and a peptide chosen from the group formed by the peptides identified by SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, derivatives thereof, pharmaceutically acceptable salts thereof and mixtures thereof.

**32.** A functional food product according to claim 31, wherein said food product comprises drinks, infusion-type foods, sauces, condiments, salad dressings, fruit juices, syrups, desserts, ice-creams, lightly frozen products, sweets or chewing gum.

**33.** A functional food product according to claim 31 or 32, further comprising one or more nutritional substances.

**34.** A functional food product according to any of claims 31 to 33, wherein said functional food product is substantially liquid.

**35.** A functional food product according to any of claims 31 to 33, wherein said functional food product is substantially solid.

**36.** A functional food product according to any of claims 31 to 33, wherein said functional food product is a gelatin.

**37.** A functional food product according to any of claims 31 to 33, wherein said food product is a dairy product.

**38.** Use of the peptides identified by SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, derivatives thereof, pharmaceutically acceptable salts thereof and mixtures thereof as a food additive.

**39.** Use of the peptides identified by SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, derivatives thereof, pharmaceutically acceptable salts thereof, and mixtures thereof as a food supplement.

**40.** Use of a method based on administering an effective amount of an antihypertensive pharmaceutical composition according to any of claims 25 to 27 to a subject chosen from a human being or an animal for the treatment and/or prophylaxis of high blood pressure in said subject, or for treating and/or preventing a disorder associated to high blood pressure, chosen form ventricular hypertrophy, congestive heart failure, cerebrovascular accidents, retinopathy, progressive renal failure, dissecting aneurysms, coronary disease and peripheral vascular diseases.

Figure 1

# Figure 2A

## CECT 5727

# Figure 2B

## CECT 5728

# Figure 2C

## CECT 5826

Fermentation time (hours)

# Figure 2D

## CECT 5827

Fermentation time (hours)

Figure 3

Figure 4

AU

Time (minutes)

# Figure 5A

Time (h)

# Figure 5B

Time (h)

# Figure 6A

# Figure 6B

# Figure 7A

# Figure 7B

# INFORME DE BUSQUEDA INTERNACIONAL

| Solicitud internacional n° |
| --- |
| PCT/ ES 2004/000227 |

## A. CLASIFICACIÓN DEL OBJETO DE LA SOLICITUD

CIP[7] C12N 1/20, A23J 3/10, 3/34, C07K 14/47, C12P 21/06, A61K 38/55, A61P 9/12 //(C12N 1/20, C12R 1:46), (C12P 21/06, C12R 1:46)

De acuerdo con la Clasificación Internacional de Patentes (CIP) o según la clasificación nacional y la CIP.

## B. SECTORES COMPRENDIDOS POR LA BÚSQUEDA

Documentación mínima buscada (sistema de clasificación seguido de los símbolos de clasificación)
CIP[7] A23J, C07K, C12P, A61K, A61P,C12R

Otra documentación consultada, además de la documentación mínima, en la medida en que tales documentos formen parte de los sectores comprendidos por la búsqueda

Bases de datos electrónicas consultadas durante la búsqueda internacional (nombre de la base de datos y, si es posible, términos de búsqueda utilizados)
CIBEPAT,EPODOC, REG, HCAPLUS

## C. DOCUMENTOS CONSIDERADOS RELEVANTES

| Categoría* | Documentos citados, con indicación, si procede, de las partes relevantes | Relevante para las reivindicaciones n° |
| --- | --- | --- |
| X | EP 1231279 A (CALPIS CO., LTD.) 14.08.2002, todo el documento, especialmente SEQ ID n° 22. | 23-39 |
| X | WO 9965326 A (NEW ZEALAND DAIRY BOARD) 23.12.1999, todo el documento, especialmente pag. 16, Tabla 3, secuencias 10,11 y reivindicaciones. | 23-39 |

☐ En la continuación del recuadro C se relacionan otros documentos    ☒ Los documentos de familias de patentes se indican en el anexo

| | |
| --- | --- |
| *   Categorías especiales de documentos citados:<br>"A"   documento que define el estado general de la técnica no considerado como particularmente relevante.<br>"E"   solicitud de patente o patente anterior pero publicada en la fecha de presentación internacional o en fecha posterior.<br>"L"   documento que puede plantear dudas sobre una reivindicación de prioridad o que se cita para determinar la fecha de publicación de otra cita o por una razón especial (como la indicada).<br>"O"   documento que se refiere a una divulgación oral, a una utilización, a una exposición o a cualquier otro medio.<br>"P"   documento publicado antes de la fecha de presentación internacional pero con posterioridad a la fecha de prioridad reivindicada. | "T"   documento ulterior publicado con posterioridad a la fecha de presentación internacional o de prioridad que no pertenece al estado de la técnica pertinente pero que se cita por permitir la comprensión del principio o teoría que constituye la base de la invención.<br>"X"   documento particularmente relevante; la invención reivindicada no puede considerarse nueva o que implique una actividad inventiva por referencia al documento aisladamente considerado.<br>"Y"   documento particularmente relevante; la invención reivindicada no puede considerarse que implique una actividad inventiva cuando el documento se asocia a otro u otros documentos de la misma naturaleza, cuya combinación resulta evidente para un experto en la materia.<br>"&"   documento que forma parte de la misma familia de patentes. |

| Fecha en que se ha concluido efectivamente la búsqueda internacional.<br>20 Julio 2004      (20.07.2004) | Fecha de expedición del informe de búsqueda internacional<br>0 3 SEP 2004      0 3. 09. 2004 |
| --- | --- |
| Nombre y dirección postal de la Administración encargada de la búsqueda internacional      O.E.P.M.<br>C/Panamá 1, 28071 Madrid, España.<br>N° de fax 34 91 3495304 | Funcionario autorizado<br>M. Novoa Sanjurjo<br><br>N° de teléfono + 34 91 3495552 |

Formulario PCT/ISA/210 (segunda hoja) (Enero 2004)

# INFORME DE BUSQUEDA INTERNACIONAL

Solicitud internacional n°

PCT/ ES 2004/000227

---

**Recuadro I Secuencia(s) de nucleótidos y/o de aminoácidos (Continuación del punto 1.b de la primera hoja)**

1.  En lo que se refiere a **las secuencias de nucleótidos y/o de aminoácidos** divulgadas en la solicitud internacional y necesarias para la invención reivindicada, la búsqueda se ha llevado a cabo sobre la base de:

   a)  Tipo de material

   ☒  una lista de secuencias

   ☐  Tabla(s) relativas a la lista de secuencias

   b)  Formato del material

   ☐  por escrito

   ☒  en soporte legible por ordenador

   c)  Fecha de presentación/entrega

   ☐  contenido en la solicitud internacional tal y como se presentó

   ☒  presentado junto con la solicitud internacional en formato legible por ordenador

   ☐  presentado posteriormente a esta Administración a los fines de la búsqueda

2.  Además, en caso de que se haya presentado más de una versión o copia de una lista de secuencias y/o tabla relacionada con ella, se ha entregado la declaración requerida de que la información contenida en las copias subsiguientes o adicionales es idéntica a la de la solicitud tal y como se presentó o no va más allá de lo presentado inicialmente.

3.  Comentarios adicionales:

Formulario PCT/ISA/210 (continuación de la primera hoja(1)) (Enero 2004)

# INFORME DE BUSQUEDA INTERNACIONAL

Solicitud internacional n°

PCT/ ES 2004/000227

---

**Recuadro II      Observaciones cuando se estime que algunas reivindicaciones no pueden ser objeto de búsqueda (Continuación del punto 2 de la primera hoja)**

De conformidad con el artículo 17.2.a), algunas reivindicaciones no han podido ser objeto de búsqueda por los siguientes motivos:

1. ☒    Las reivindicaciones n°s: 9-11, 40
      se refieren a un objeto con respecto al cual esta Administración no está obligada a proceder a la búsqueda, a saber:
   Aunque dichas reivindicaciones se refieren a un método de tratamiento aplicado directamente al cuerpo humano o animal, la búsqueda se ha realizado en relación a los efectos del compuesto/composición. Regla 39.1.iv PCT.

2. ☐    Las reivindicaciones n°s:
      se refieren a elementos de la solicitud internacional que no cumplen con los requisitos establecidos, de tal modo que no pueda efectuarse una búsqueda provechosa, concretamente:

3. ☐    Las reivindicaciones n°s:
      son reivindicaciones dependientes y no están redactadas de conformidad con los párrafos segundo y tercero de la regla 6.4.a).

---

**Recuadro III    Observaciones cuando falta unidad de invención (Continuación del punto 3 de la primera hoja)**

La Administración encargada de la Búsqueda Internacional ha detectado varias invenciones en la presente solicitud internacional, a saber:
   Invención 1.- Cepas de Enterococcus faecalis, péptidos de SEQ ID n°2,3,4,6 y 7, y aplicaciones.
   Invención 2.- Empleo de cepas de Enterococcus faecalis en la obtención del péptido SEQ ID n° 1.
   Invención 3.- Empleo de cepas de Enterococcus faecalis en la obtención del péptido SEQ ID n° 5.
   Invención 4.- Empleo de cepas de Enterococcus faecalis en la obtención del péptido SEQ ID n° 8

1. ☐    Dado que todas las tasas adicionales han sido satisfechas por el solicitante dentro del plazo, el presente informe de búsqueda internacional comprende todas las reivindicaciones que pueden ser objeto de búsqueda.

2. ☒    Dado que todas las reivindicaciones que pueden ser objeto de búsqueda pueden serlo sin un esfuerzo particular que justifique una tasa adicional, esta Administración no ha invitado al pago de ninguna tasa de esta naturaleza

3. ☐    Dado que tan sólo una parte de las tasas adicionales solicitadas ha sido satisfecha dentro del plazo por el solicitante, el presente informe de búsqueda internacional comprende solamente aquellas reivindicaciones respecto de las cuales han sido satisfechas las tasas, concretamente las reivindicaciones n°s:

4. ☐    Ninguna de las tasas adicionales solicitadas ha sido satisfecha por el solicitante dentro de plazo. En consecuencia, el presente informe de búsqueda internacional se limita a la invención mencionada en primer término en las reivindicaciones, cubierta por las reivindicaciones n°s:

**Indicación en cuanto a la protesta**        ☐  Las tasas adicionales han sido acompañadas de una protesta por parte del solicitante.

☐  El pago de las tasas adicionales no ha sido acompañado de ninguna protesta.

Formulario PCT/ISA/210 (continuación de la primera hoja (2)) (Enero 2004)

## INFORME DE BUSQUEDA INTERNACIONAL

Información relativa a miembros de familias de patentes

Solicitud internacional n°

PCT/ ES 2004/000227

| Documento de patente citado en el informe de búsqueda | Fecha de publicación | Miembro(s) de la familia de patentes | Fecha de publicación |
|---|---|---|---|
| EP 1231279 A | 14.08.2002 | WO 0134828 A | 17.05.2001 |
| | | JP 2001136995 A | 22.05.2001 |
| | | AU 1306001 A | 06.06.2001 |
| | | EP 20000974904 | 10.11.2000 |
| | | CN 1423704 T | 11.06.2003 |
| | | NZ 518957 A | 29.08.2003 |
| | | AU 773587 B | 27.05.2004 |
| WO 9965326 A | 23.12.1999 | AU 4535999 A | 05.01.2000 |
| | | NO 20006305 A | 14.02.2001 |
| | | EP 1087668 A | 04.04.2001 |
| | | EP 19990928257 | 14.06.1999 |
| | | AU 761477 B | 05.06.2003 |
| | | NZ 508867 A | 28.11.2003 |
| | | US 2004086958 A | 06.05.2004 |

Formulario PCT/ISA/210 (anexo_familia de patentes) (Enero 2004)

# EP 1 640 447 A1

## INTERNATIONAL SEARCH REPORT

International application No.

PCT/ ES 2004/000227

**A. CLASSIFICATION OF SUBJECT MATTER**

**IPC 7** C12N 1/20, A23J 3/10, 3/34, C07K 14/47, C12P 21/06, A61K 38/55, A61P 9/12 //(C12N 1/20, C12R 1:46), (C12P 21/06, C12R 1:46)

According to International Patent Classification (IPC) or to both national classification and IPC

**B. FIELDS SEARCHED**

Minimum documentation searched (classification system followed by classification symbols)

**IPC 7** A23J, C07K, C12P, A61K, A61P,C12R

Documentation searched other than minimum documentation to the extent that such documents are included in the fields searched

Electronic data base consulted during the international search (name of data base and, where practicable, search terms used)

**CIBEPAT,EPODOC, REG, HCAPLUS**

**C. DOCUMENTS CONSIDERED TO BE RELEVANT**

| Category* | Citation of document, with indication, where appropriate, of the relevant passages | Relevant to claim No. |
|---|---|---|
| X | EP 1231279 A (CALPIS CO., LTD.) 14.08.2002, **the whole document, particularly SEQ ID n° 22** | 23-39 |
| X | WO 9965326 A (NEW ZEALAND DAIRY BOARD) 23.12.1999, **the whole document, particularly page 16, table 3, sequences 10, 11 and claims** | 23-39 |

☐ Further documents are listed in the continuation of Box C.     ☒ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| **20 JULY 2004 (20.07.04)** | 0 3 SEP 2004     0 3. 09. 2004 |

| Name and mailing address of the ISA/ **S.P.T.O.** | Authorized officer |
|---|---|
| Facsimile No. | Telephone No. |

Form PCT/ISA/210 (second sheet) (July 1992)

38

## INTERNATIONAL SEARCH REPORT

| International application No. |
| --- |
| PCT/ ES 2004/000227 |

---

**Box I    Observations where certain claims were found unsearchable (Continuation of item 1 of first sheet)**

This international search report has not been established in respect of certain claims under Article 17(2)(a) for the following reasons:

1. [x] Claims Nos.: 9-11 and 40
because they relate to subject matter not required to be searched by this Authority, namely:

Although claims 9-11 and 40 relate to a method of treatment performed directly on the human or animal body, the search was carried out in relation to the effects of the compound or composition (PCT Rule 39.1(iv)).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

---

**Box II    Observations where unity of invention is lacking (Continuation of item 2 of first sheet)**

This International Searching Authority found multiple inventions in this international application, as follows:

Invention 1: Strains of *Enterococcus faecalis*, peptides with SEQ ID
Nos 2, 3, 4, 6 and 7 and applications.
Invention 2: Use of strains of *Enterococcus faecalis* for obtaining the
peptide with SEQ ID No. 1.
Invention 3: Use of strains of *Enterococcus faecalis* for obtaining the
peptide with SEQ ID No. 5.
Invention 4: Use of strains of *Enterococcus faecalis* for obtaining the
peptide with SEQ ID No. 8.

1. [ ] As all required additional search fees were timely paid by the applicant, this international search report covers all searchable claims.

2. [x] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**    [ ] The additional search fees were accompanied by the applicant's protest.
[ ] No protest accompanied the payment of additional search fees.

Form PCT/ISA/210 (continuation of first sheet (1)) (July 1992)

**EP 1 640 447 A1**

## INTERNATIONAL SEARCH REPORT

International application No.

**PCT/ ES 2004/000227**

**Recuadro I Secuencia(s) de nucleótidos y/o de aminoácidos (Continuación del punto 1.b de la primera hoja)**

1. En lo que se refiere a **las secuencias de nucleótidos y/o de aminoácidos** divulgadas en la solicitud internacional y necesarias para la invención reivindicada, la búsqueda se ha llevado a cabo sobre la base de:

    a) Tipo de material

        ☒ una lista de secuencias

        ☐ Tabla(s) relativas a la lista de secuencias

    b) Formato del material

        ☐ por escrito

        ☒ en soporte legible por ordenador

    c) Fecha de presentación/entrega

        ☐ contenido en la solicitud internacional tal y como se presentó

        ☒ presentado junto con la solicitud internacional en formato legible por ordenador

        ☐ presentado posteriormente a esta Administración a los fines de la búsqueda

2. Además, en caso de que se haya presentado más de una versión o copia de una lista de secuencias y/o tabla relacionada con ella, se ha entregado la declaración requerida de que la información contenida en las copias subsiguientes o adicionales es idéntica a la de la solicitud tal y como se presentó o no va más allá de lo presentado inicialmente.

3. Comentarios adicionales:

Form PCT/ISA/210

40

## INTERNATIONAL SEARCH REPORT
Information on patent family members

International Application No

PCT/ ES 2004/000227

| Patent document cited in search report | Publication date | Patent familiy member(s) | Publication date |
|---|---|---|---|
| EP 1231279 A | 14.08.2002 | WO 0134828 A | 17.05.2001 |
| | | JP 2001136995 A | 22.05.2001 |
| | | AU 1306001 A | 06.06.2001 |
| | | EP 20000974904 | 10.11.2000 |
| | | CN 1423704 T | 11.06.2003 |
| | | NZ 518957 A | 29.08.2003 |
| | | AU 773587 B | 27.05.2004 |
| WO 9965326 A | 23.12.1999 | AU 4535999 A | 05.01.2000 |
| | | NO 20006305 A | 14.02.2001 |
| | | EP 1087668 A | 04.04.2001 |
| | | EP 19990928257 | 14.06.1999 |
| | | AU 761477 B | 05.06.2003 |
| | | NZ 508867 A | 28.11.2003 |
| | | US 2004086958 A | 06.05.2004 |

Form PCT/ISA/210 (patent family annex) (July 1992)